# EUROPEAN PATENT SPECIFICATION

(11) **EP 1 673 113 B1**
(45) Date of publication and mention of the grant of the patent: **29.12.2010**
(21) Application number: 04788759.1
(22) Date of filing: 14.09.2004
(51) Int. Cl.: A61L 27/28, A61L 27/54

(54) **IMPLANTS WITH ATTACHED SILYLATED THERAPEUTIC AGENTS**
IMPLANTATE MIT BEFESTIGTEN SILYLIERTEN THERAPEUTISCHEN MITTELN
IMPLANTS AYANT LES AGENTS THERAPEUTIQUES SILYES

(30) Priority: 15.09.2003 US 662261
(43) Date of publication of application: 28.06.2006
(73) Proprietor: Smart Tech LP, King of Prussia PA 19406 (US)
(72) Inventor: Wickstrom, Eric, Philadelphia, PA 19130 (US); Hickok, Noreen J., Philadelphia, PA 19130 (US); Parvizi, Javad, Gladwyne, PA 19035 (US)
(74) Representative: Katzameyer, Michael
(86) International application number: PCT/US2004/030096
(87) International publication number: WO 2005/027990

(56) References cited:
- WO-A-99/26674
- WO-A-03/008006
- FERRIS D M ET AL: "RGD-coated titanium implants stimulate increased bone formation in vivo" BIOMATERIALS, ELSEVIER SCIENCE PUBLISHERS BV., BARKING, GB, vol. 20, no. 23-24, December 1999 (1999-12), pages 2323-2331, XP002288743 ISSN: 0142-9612
- REZANIA ALIREZA ET AL: "Bioactivation of metal oxide surfaces. 1. Surface characterization and cell response" LANGMUIR; LANGMUIR 1999 ACS, WASHINGTON, DC, USA, vol. 15, no. 20, 1999, pages 6931-6939, XP002316733

## Description

### BACKGROUND AND SUMMARY OF THE INVENTION

Implant-associated infections are an ever-present and devastating complication of insertion of a foreign object, such as a stent, catheter, intravenous delivery tube (Hickman), heart valve, dental implant, electro-mechanical device, prosthetic device, glucose sensor, or stabilizing device such as orthopedic nails and pins. Sources of these infections include introduction of foreign bodies during wounding, introduction of microorganisms in the surgical suite, constant access to indwelling catheters, and hematogenous infections that arise at a site distal from the implant. Generally, infections associated with implants currently require systemic pharmaceutical treatment of the patient.

Open fracture wounds from ballistic injury are also a serious health problem and require on-site external fixation followed by internal fixation to allow the fracture to heal. In such wounds, the probability of infection is very high. The extended infection control procedures and subsequent protracted healing time results in prolonged disability, often incomplete healing, and hospital costs.

Generally, principles of open fracture management include emergent and adequate wound debridement, often on multiple occasions, immobilization (often by external fixation), antibiotic therapy, and secondary closure or coverage of the wound. The definitive fracture management by intramedullary nail insertion is delayed until adequate soft tissue repair has occurred. Open fractures in general, and high-energy gunshot wounds, in particular require immediate and aggressive irrigation and debridement for removal of microscopic and macroscopic contaminants such as clothing and bullet wadding. Nevertheless, despite immediate surgical intervention open wounds often become infected. In animal studies as many as 60% of wounds can be infected within 12 hours of such trauma. Importantly, the early administration of antibiotics inhibits bacterial growth associated with delays in debridement, while reducing subsequent tissue necrosis.

Periprosthetic infections after orthopaedic surgery are extremely costly and frequently lead to prolonged disability. Improvements in sterile technique have lowered the incidence to below 1% in many high throughput centers. However, cases of post-surgical infections still are numerous. Each case requires a minimum of aggressive antibiotic treatment and often at least one additional surgery. Unfortunately, for the population most frequently affected by such infections, i.e. the elderly, the diabetic, and the immunocompromised, such surgeries place the patient at great risk.

In general, implant-associated infections arise from two causes. The first of these, the so-called hematogenous infection, is due to an infection that is present elsewhere in the body. Bacteria from this infection opportunistically populate the protected environment around the partially encapsulated/partially integrated implant. The second cause of infections is the introduction of microorganisms during surgery. To minimize introduction of infection, very stringent conditions have been developed for the surgical site, which include use of space suits, filtered air, and laminar flow areas. Additionally, there is constant lavage of the surgical site with antibiotic solutions and post-surgical systemic prophylaxis.

Irrespective of the etiology, the patient can be aggressively treated with antibiotics and/or surgery. For a periprosthetic infection, the surgery minimally involves debridement of the bone to remove adherent microorganisms and replacement of the polymer parts. More aggressive procedures are employed in many circumstances, including placement of a spacer block that can deliver therapeutic doses of antibiotic to stabilize the cavity or use of antibiotic-releasing cement to anchor the prosthesis. Commonly, these regimens result in a compromised bone stock; in recalcitrant infections, arthrodesis or amputation may be required. Thus, any approach to limit or even prevent implant-associated infections in general and orthopaedic infections in particular can have a large impact on reducing healthcare costs, minimizing pain and suffering, and improving the quality of life of the affected patients.

Surgical implantation of a prosthesis results in formation of a fibrous clot, followed by fibrous encapsulation of the foreign object with varying degrees of inflammation. Subsequently, as much as 90% of the implant is surrounded by a fibrous membrane. Unfortunately, the encapsulation by the fibrous membrane provides a protected microenvironment that is ideal for bacterial propagation. Initial stages of bacterial colonization involve the switch from a mobile, planktonic bacterium to one that is adherent to the inert surface. As the numbers of bacteria become greater, they organize into colonies that can form large biofilms. These sites on the implant surface are poorly accessed by the immune system and are chemically protected from antibiotics.

The majority of periprosthetic infections are caused by *Staphylococcus aureus,* and *S. epidermidis,* both gram-positive bacteria; less frequent infections are caused by the gram-negative organisms. Both *S. aureus* and *S. epidermidis* are commonly present in the operating room environment and are implicated in infections involving prostheses, stents and other biomaterial implants. Both species adhere to the biomaterial surfaces, propagate rapidly, and during this proliferation, generate a pre-biofilm slime. Production of the polysaccharide-enclosed clumps of bacteria, characteristic of a biofilm, completes the process of restricting antibiotic access to the bacterial surface. This biofilm can effectively immobilize many antibiotics thereby reducing the numbers of therapeutic molecules that can penetrate and interact with the bacteria. Both *S. aureus* and *S. epidennidis* form such biofilms. When biofilms are formed, surgical experience dictates complete removal of the prosthetic components and debridement. Clearly, if antibiotics can be released at an early colonization or pre-colonization stage, the biofilm formation can be impeded and the infection can be extinguished, obviating the need for surgical intervention.

Infection following joint arthroplasty is a devastating complication with immense financial and psychological costs. Effective measures, including the use of body exhaust systems, laminar airflow, prophylactic antibiotics, and various other precautions, have been successful in reducing the incidence of periprosthetic infection. Despite all these measures, deep infection still occurs after 1-5 percent of joint replacements. The incidence is even higher in some 'at risk' patients such as diabetics, patients with remote history of infection, and those with inflammatory arthropathies. Generally, principles of periprosthetic infection management include emergent debridement and irrigation in select cases, resection arthroplasty and delayed reimplantation until eradication of the organisms has occurred. Prolonged antibiotic treatment in concert with multiple surgeries results in extended periods of patient disability. A multitude of factors influence the success of these surgical interventions. The most important relate to the efficacy by which antibiotics are delivered to the local tissues and the osteogenic potential of the surrounding bone.

Infection prevention/treatment in patients with implants requires robust regiments that may include systemic antibiotic treatment and use of implants that achieve a controlled antibiotic release, usually in the immediate post-operative period. It would be desirable to provide for an implant that maintains a reservoir of antibiotics or other therapeutic molecules that can be activated at the time of an establishing infection, whether in the immediate post-operative period or at longer times after implantation.
WO 99/26674 and WO 03/008006 disclose titanium implants having bioactive surfaces. The linkers used in these documents differ from those of the present invention.

### SUMMARY

The present invention is an implant as defined by claim 1 that includes therapeutic molecules like antibiotics bonded to the implant surface and that interact with cells that are adjacent, near, or adhering to the implant. The therapeutic molecules may be bonded to the implant surface by an acid-labile linkage; a covalent linkage that includes a cell membrane-soluble portion between the implant and the tethered therapeutic molecule; a covalent linkage connected to a ligand that is then loaded with the therapeutic molecule through its high affinity binding to its ligand, and/or an enzyme-labile linkage. Enzymes, acids, or endogenous ligands characterizing the cells or their activities may interact with or cleave the bonded therapeutic molecules on the implant. The therapeutic molecules may be used to alter the proliferation of these cells and the implant may be used to promote the adhesion and maturation of these cells. The surface modified implant provides a reservoir of therapeutic molecules that are controllably released in response to the cellular environment of the implant.

One embodiment of the present invention is an implant for a mammal. The implant has a biologically compatible surface with at least a portion of the surface silylated with organosilanes. One or more of these organosilane may be bonded to therapeutic molecules with the therapeutic molecule interacting with cells adjacent to the surface of the implant. The bond between the organosilane and the therapeutic molecules may be a covalent bond, and can include an acid-labile or enzyme-labile bond such that the therapeutic molecule is released from the organosilane by interacting with enzymes or acids produced by the cells. The therapeutic molecule may also be competitively bonded to the organosilane and released by exchange with endogenous ligands of the cell. The therapeutic molecules may include peptides, therapeutic oligonucleotides, antibiotics, cell growth factors, chemotherapeutics, thrombolytic, anti-inflammatories, osteoactive factors and combinations of these molecules. The therapeutic molecules covalently bonded to the implant may be used to alter angiogenesis, or decrease bacterial proliferation in cells or tissue adjacent to the implant. Preferably the bioactive surface remains active until the danger of disease has passed.

One embodiment of the present invention is an implant for a mammal having a biologically compatible surface. At least a portion of the surface is silylated with organosilanes and one or more of the organosilanes is covalently bonded to a first end of a linking group. The second end of the linking group may be bonded to therapeutic molecules that interact with cells adjacent to the surface of the implant. The therapeutic molecule may include peptides, therapeutic oligonucleotides, antibiotics, cell growth factors, chemotherapeutics, anti-thrombolytic, anti-inflammatories, osteoactive factors, and combinations of these. The therapeutic molecule may be bonded to a linking group that can enter or pass through the membrane or the wall of cells. The linker bonded to the organosilane can include amino acids, a peptide, or spacers like oligo(ethylene glycol). The linker may also include an acid labile moiety methylmaleamide, hydrazone, and combinations of these. Labilization of covalently bonded therapeutic molecules from the linker by cellular acid or enzymes releases the therapeutic molecule from the implant to interact with the cells and preferably leaves a peptide covalently bonded to the surface that may be used to promote the adhesion and maturation of cells on the implant. The therapeutic molecule can be competitively bonded to the linker and may be exchanged with endogenous ligands of the cell. The therapeutic molecules bonded to the implant may interact with the cells to alter angiogenesis, or decrease bacterial proliferation adjacent to the implant.

One embodiment of the present invention is an implant for use in a mammal that has a first terminal end of one or more linking groups covalently bonded to a silylated implant surface. The second, or opposite, terminal end of the one or more of the linking groups can be bonded to an antibiotic that is used to interact with cells adjacent to the surface of the implant. The linking group bonded to the implant surface preferably includes an integrin binding peptide sequence and can include an acid or enzyme-labile bond such that labilization of the antibiotic molecule from the linker provides a peptide that promotes the adhesion and maturation of bone cells. The antibiotic may be tethered to the implant through a long, flexible partially membrane-soluble linker or the antibiotic may be competitively bonded to the linker and released by competitive binding with endogenous ligands of the cell. The antibiotic may be active against gram negative bacteria, active against both gram-positive and gram-negative bacteria, and preferably the antibiotic is active against gram positive bacteria. The antibiotic may include but is not limited to minocyclins, tigecyclin, glycylcycline, vancomycin and its analogs, rifampin and its analogs, gentamycin and its analogs, or combinations thereof.

One embodiment of the present invention is a method of treating a mammal that includes inserting an implant into a site such as a fracture, an artery, or body cavity of the mammal. The implant has a biologically compatible surface that is silylated with organosilanes and where one or more of the organosilanes is bonded to therapeutic molecules. The therapeutic molecule interacts with cells adjacent to the surface of the implant in the mammal. The therapeutic molecule may remain bonded to the implant and interact with the cells or they can be released from the organosilane by reaction with a cellularly derived acid, enzyme, or ligand. The therapeutic molecules from the implant can alter the proliferation of cells at the site of the implant. Preferably the implant promotes osseointegration during fracture fixation and the therapeutic molecules bonded to the implant include antibiotics to prevent bacterial proliferation at a fracture fixation site. The surface-modified implant, by virtue of the tethered antibiotics, can be used to inhibit bacterial proliferation after the initial adhesion of the bacterium to the implant surface in a mammalian patient.

In a preferred embodiment antibiotics can be covalently bonded or complexed to a metal such as titanium by organosilane linkers that have sites or moieties that are degraded enzymatically or are labile under various pH conditions. In a preferred embodiment, agents released from bacteria near or adhering to an implant can cleave an antibiotic-linker bond so as to release high focal concentrations of an antibiotic from the implant, killing susceptible bacteria. Even more preferably, the surface modifications on the implant lower bacterial counts while sustaining osteoblastic cell proliferation.

The overall activity of these modified implant surfaces can be tailored so that they react at the earliest stages of infection as the bacteria begin to colonize the implant surface. Preferably the adherent bacteria encounters a surface that can either release antibiotic from the linker due to local pH changes associated with the proliferating bacteria, release antibiotic by linker cleavage due to cellular enzyme release, or release antibiotic by competition from a linkage by an endogenous ligand of the cell. Alternatively, the cells may internalize the antibiotic because of the membrane-solubility of a linker. This internalized antibiotic may be active while still bound to the implant, as is the case for vancomycin. Alternatively, where the enzyme to cleave the bioactive molecule resides in the bacterial wall, in the cell membrane, or in the cellular milieu, an enzyme associated with the bacteria or with the cell cleaves the bioactive moiety from the linker to release it within the cell. This directed release of antibiotic would result in preferential killing of adherent bacteria, i.e. those that are colonizing the surface to initiate biofilm formation.

The release of therapeutic molecules such as antibiotics from the modified implant surface occurs as a function of bacterial colonization and thus the bioactive moieties remain latent until activated by the presence of bacteria. Such a device can be used for the prevention of implant-related infections, both immediately after the implant is inserted as well as for the longer term prevention of hematogenous infections.

By prevention of bacterial colonization of the implant surface, any remaining bacteria are accessible for clearance by the immune system. The implant surface modifications of the present invention are inert under conditions where their activity is not required and are active when bacteria begin to foul an implant. It has a clear advantage over the existing technologies in that the surface does not release active antibiotics until cued to do so by bacterial adhesion. Moreover, these modified implants can be formulated to be stable for long periods, thus having a ready reservoir of antibiotics to reduce the risks of secondary infections. The surface-modified implants of the present invention may also be made to include other therapeutic molecules tethered to the surface for the treatment of conditions such as cancer, restenosis, bone loss, and thromboses.

One use for the present invention is for the modification of implant surfaces with antibiotics that are most frequently fouled by biofilm formation, such as prosthesis and nails used for fracture fixation and surfaces of indwelling catheters. The bactericidal surface could also be used to modify implant surfaces in less high-risk implant situations, such as the orthopaedic arthroplasties and stent placement where infection is infrequent, but once established can have devastating consequences.

### DESCRIPTION OF THE DRAWINGS

In part, other aspects, features, benefits and advantages of the embodiments of the present invention can be apparent with regard to the following description, appended claims and accompanying drawings where:

FIG. 1 Illustrates the general chemical structure of an implant surface modified by covalent bonding of molecules to its surface;

FIG. 2 MALDI-TOF mass spectroscopy of putative VAN-AEEA-AEEA-NH-Pr-Ti6A14V from a 2,5-dihydroxy-benzoic acid (2,5-DHBA) matrix;

FIG. 3 Ti-tethered vancomycin kills adherent *S. aureus.* Vancomycin, tethered to the Ti surface, was incubated with *S. aureus* for 1 h and stained for bacterial viability. Live bacteria stain green (light gray)-note the small number of live bacteria on the vancomycin tethered surface (C) (VAN-OEG-APTS-Ti). Dead bacteria stain red (light gray); note the larger number of dead bacteria on the vancomycin-tethered to the Ti surface (D);

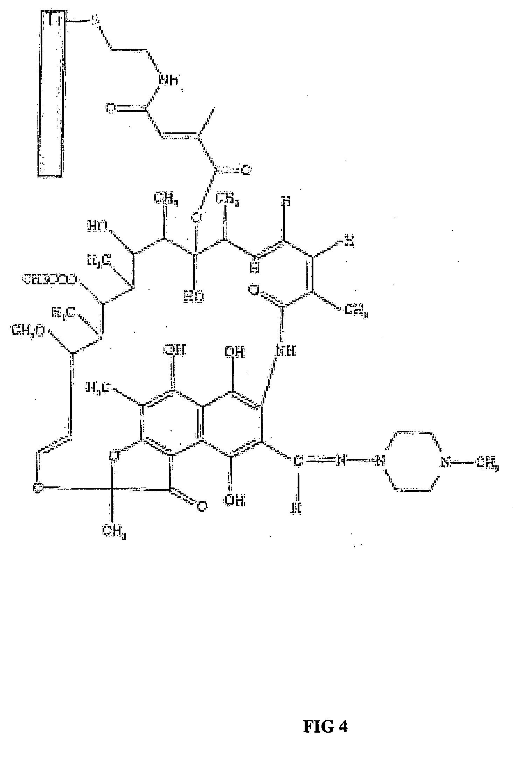

FIG. 4A schematic diagram of the antibiotic 17-(2-methyl-4-thioethylamido maleyl) rifampin modified at 17-OH for bonding with an implant surface silylated with MPTS to form a disulfide or GPTS to form a thioether;

FIG. 5(A) is a schematic illustration of the bioactive molecule vancomycin, whose linkage to the metal implant is labile to acid, reacting with acid secreted by bacterium near the implant surface; (B) illustrates the released vancomycin from the modified implant interacting with the bacterium;

FIG. 6(A) is a schematic illustration of the bioactive molecule vancomycin irreversibly tethered by a covalent linkage to the implant surface; (B) illustrates the passage of the bioactive molecule through the cell wall using the membrane soluble linkage that places vancomycin at its site of action in the cell;

FIG. 7(A) is a schematic illustration of the bioactive molecule vancomycin bound to an affinity ligand connected to the implant surface; (B) illustrates the vancomycin is released by competitive binding with its native substrate in the bacterium;

FIG. 8 Illustrates the chemical structure of linkages bonded to the implant surface represented by R₁ and the acid labile moieties maleamide and hydrazone; groups R₂ and R₃ represent therapeutic molecules;

FIG. 9(A) is a schematic illustration of the antibiotic tigecyclin linked to Ti-RGD (SEQ ID NO: 1) by acid-labile linkages that are cleaved by acid secreted by an adjacent bacterium; (B) illustrates released antibiotic interacting with the bacterium and that the released tigecyclin has exposed the RGD (SEQ ID NO: 1) motif bonded to the implant surface;

FIG. 10(A) is a schematic illustration of a modified implant surface with covalently bonded acid-labile tigecyclin bioactive therapeutic molecules bonded to a portion of the RGD (SEQ ID NO: 1) peptides linked to the implant surface; (B) is a schematic illustration of the modified surface releasing tigecyclin in response to acid released by a cell; (C) is a schematic illustration of the modified implant surface illustrating osteoblast cell attachment, integrin clustering, and extracellular matrix protein deposition.

### DETAILED DESCRIPTION OF THE INVENTION

Before the present compositions and methods are described, it is to be understood that this invention is not limited to the particular molecules, compositions, methodologies or protocols described, as these may vary. It is also to be understood that the terminology used in the description is for the purpose of describing the particular versions or embodiments only, and is not intended to limit the scope of the present invention that can be limited only by the appended claims.

It must also be noted that as used herein and in the appended claims, the singular forms "a", "an", and "the" include plural reference unless the context clearly dictates otherwise. Thus, for example, reference to a "cell" is a reference to one or more cells and equivalents thereof known to those skilled in the art, and so forth. Unless defined otherwise, all technical and scientific terms used herein have the same meanings as commonly understood by one of ordinary skill in the art. Although any methods and materials similar or equivalent to those described herein can be used in the practice or testing of embodiments of the present invention, the preferred methods, devices, and materials are now described. All publications mentioned herein are incorporated by reference. Nothing herein is to be construed as an admission that the invention is not entitled to antedate such disclosure by virtue of prior invention.

The present invention is directed to bonding therapeutic molecules to the surface of implantable substrates that used for fracture fixation, prosthetics, catheters, or monitoring sensors in mammals. The therapeutic molecules may be bonded to the implant surface by linkages that release the molecules in response to cellular activity by the cells near the modified implant surface.

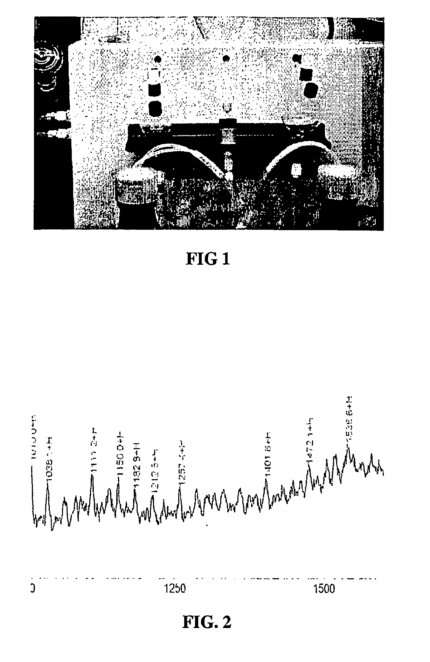

A schematic diagram of a non-limiting embodiment of the present invention where a therapeutic molecule is bonded to the surface of an implant is shown in FIG. 1. The implant surface is silylated using an organosilane which has an organic group that may be used to couple other molecules to the implant surface. The organic group may include but is not limited to one or more amine groups, epoxides, carboxylic acids, or thiol groups. Silylation of the implant is represented by the Ti-O-Si (CH₂)₃NH portion (A) of the structure in FIG. 1. The first end or terminus of a linking group is covalently bonded to the organic group of the organosilane. As shown in FIG. 1, this may be coupling of the amino end of the organosilane with the carboxyl end of a representative linker with a cell adhesion sequence that includes amino acids forming the peptide sequence RGDS (SEQ ID NO:2), portion (B). Other linkers may include different amino acids, the use different sized flexible oligo-ethylene glycol spacers, or a different reactive group at the terminal ends of the linking group. The second or opposite terminus end of the representative linker, in FIG. 1 it is an amino end however other groups may be used, is bonded to a representative labile linkage. In FIG. 1 the acid-labile methylmaleamide linkage moiety is shown as portion (C). Other labile linkages may be used including but not limited to enzyme-labile linkages. A representative therapeutic molecule (D), tigecyclin in FIG. **1**, is bonded to the acid-labile moiety portion (C). This therapeutic molecule interacts with cells adjacent to the surface of the implant. Although FIG. 1 illustrates various portions (B), (C), and (D) of the modified titanium implant surface, the present invention is not limited to this structure. For example the segments (B), (C) and (D) may be covalently linked together as a single unit and coupled to the silylated implant surface. Alternatively, the labile group may be coupled directly to the organosilane or the therapeutic molecule might be covalently bonded directly to an organosilane and this molecule used to silylate the surface of the implant.

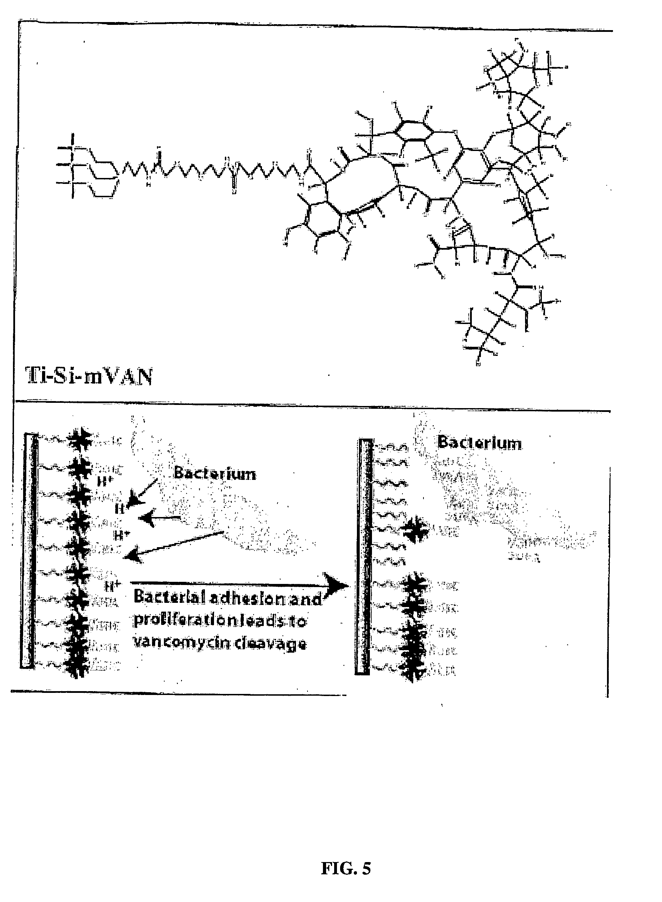

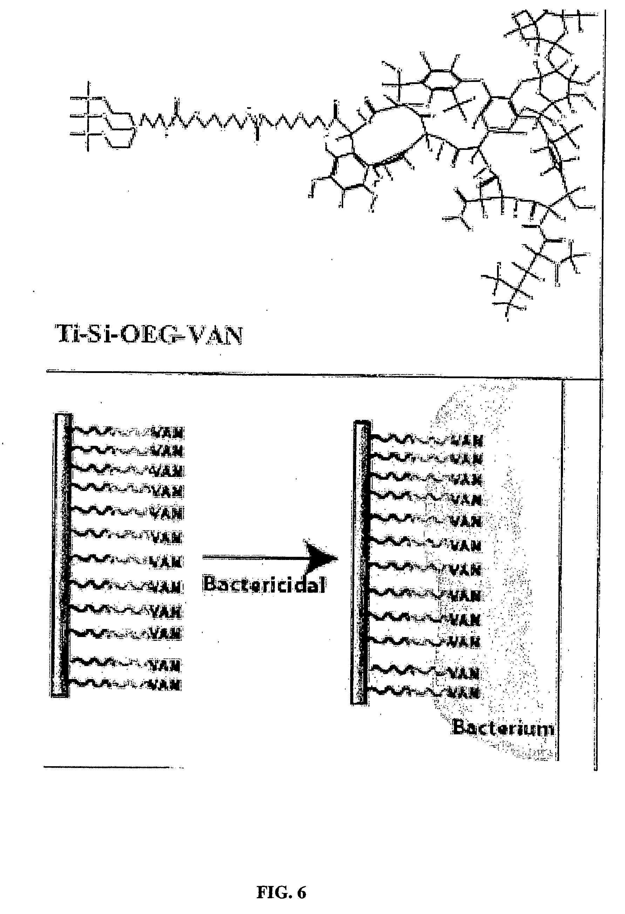

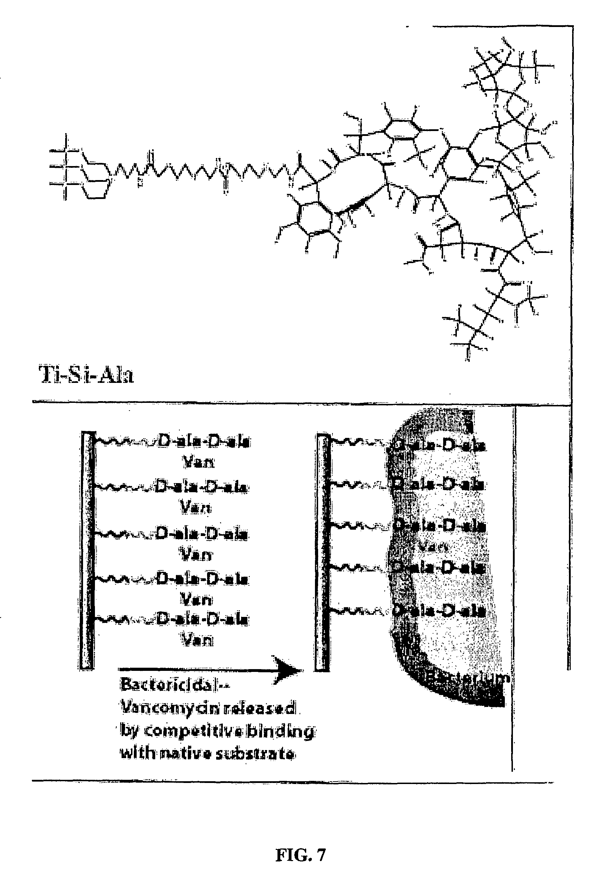

In embodiments of the present invention, therapeutic molecules are bonded to the surface of an implant. The bonds connecting the linker and organosilane to the implant surface are preferably covalent bonds. The therapeutic molecules may be bonded to the linker or organosilane through covalent or competitive bonding. The bond between the implant surface and the therapeutic molecule may include a labile bond from a linker to the therapeutic moiety. This bond may be cleaved by a secretion by a cell which would include acids and enzymes. The therapeutic molecule would be released in the immediate vicinity of the cell that released the secretion to target its activity to that cell as shown schematically in FIG. 5 where the antibiotic vancomycin is tethered to a Ti surface through an acid-labile linker. A stable non-labile bond from the linker to the therapeutic moiety, such as an oligo(ethyleneglycol) chain spacer, would allow the therapeutic molecule to enter or pass through the cell wall or cell membrane to be presented into the cellular space. In this case the therapeutic moiety remains active in its tethered state, such as for example the antibiotic vancomycin as shown schematically in FIG. 6. Alternatively, the therapeutic molecule may be competitively bonded to linking groups or organosilanes that are covalently bound to the surface of the implant. The therapeutic molecule competitively bonded to the linking groups or organosilanes may then pass through a cell wall or membrane. Endogenous ligands of the cell competitively bind to the linking groups or organosilanes releasing the therapeutic molecule within the cell as illustrated schematically for vancomycin in FIG. 7. The surface modified implant may also include a linker or organosilane with a labile bond that is adjacent to the therapeutic molecule and that is capable of spanning a cell wall or cell membrane. The labile bond of the linker would be cleaved by intracellular enzymes, thereby releasing the drug or peptide within the cell allowing it to have its specific effect. A non-limiting example of such an intracellular enzyme would be a kinase inhibitor.

The therapeutic molecule may be tethered or bonded to the implant surface through an organosilane or through a linking group bonded to the organosilane. The linker or organosilane may have an acid-labile site, an enzyme cleavage site, recognition sequences for metalloproteinases, plasmin, thrombin, or tissue plasminogen activator. The linker or organosilane can be a long flexible partially membrane soluble chain that is covalently bonded directly to the therapeutic molecule. For example, organosilane groups may be coupled to oligo(ethylene glycol) linkers on the implant surface and the other end of the linker used to covalently bond to a therapeutic molecule such as vancomycin as shown in FIG. 6. Alternatively the flexible partially membrane soluble chain may release the therapeutic molecule within the cell through a high affinity competitively bonded tethered ligand such as but not limited to sequences such as (D-Ala-D-Ala) as shown in FIG. 7, or by incorporating an enzyme cleavage site into the flexible chain. Cleavage of acid-labile or enzyme-labile linkers and organosilanes to release the therapeutic molecules can expose a peptide sequence, such as RGD (SEQ ID NO: 1), which supports cellular proliferation.

Based on a therapeutic molecule such as an antibiotic occupying -40 nm² of space, if an adherent bacterium or other cell occupies 1 µm, it could have ~25,000 therapeutic molecules of antibiotic tethered for release directly at the bacterium's surface. The concentration of therapeutic molecules tethered to the implant surface may be increased by bonding the bioactive molecule to the interstices of a porous implant surface or within the interconnected regions to allow both increased effects of local concentrations, increased therapeutic molecule capacity, as well as increased surface area for the additional therapeutic molecules. Multi-functional linkages may also be used to tether more than one therapeutic molecule per site. Such branched linkers can serve to tether multiple antibiotic or other therapeutic molecules to the implant surface through a single bond between the surface and the linker.

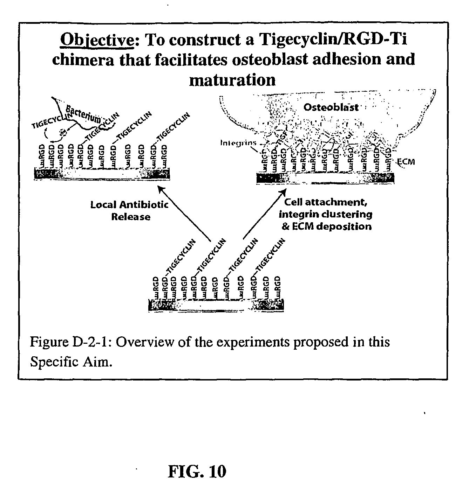

The linker or organosilane may be a flexible membrane soluble chain such as but not limited to oligo-ethylene glycol (OEG), and may also include naturally occurring and non-naturally occurring amino acids and peptide sequences including such amino acids. Oligo-ethylene glycol terminated surfaces exhibit resistance to protein absorption and may have from 2 to 20 repeat units. As shown schematically in FIG. 10, a silane linkage may be used to tether or bond adhesion-enhancing peptides such as RGD (SEQ ID NO: 1) to a Ti containing implant substrates that are linked to therapeutic molecules like tigecyclin. The one or more therapeutic molecules modifying the surface of the implant may be bonded to all or a portion of the surface of the implant through the linkers or the organosilane. One or more different linkages or organosilane may be used on different portions of the implant surface and mixtures of linkages and organosilanes may be used to modify the implant surface as well. The linkages and organosilane may include amino acid and peptide sequences which interact with cells, for example but not limited to RGD (SEQ ID NO: 1), and RGDS (SEQ ID NO: 2), or they may include non-peptide linkages such as AEEA, polyethylene glycol (PEG), and OEG. Additional peptide sequences useful as linkers in the present invention include those used for ECM peptidomimics such as RGES (SEQ ID NO: 3), DGEA (SEQ ID NO: 4), and EGEA (SEQ ID NO: 5) as well as the bioactive peptides disclosed in U.S. Pat. No. 6,428,579.

The therapeutic molecule can be but is not limited to peptides, therapeutic oligonucleotides, or molecules like antibiotics, anitfungals, thrombolytics, anti-inflammatories, osteoactive factors, or chemotherapeutics. The therapeutic molecule should remain attached to the implant surface in the physiological range near 7.4 for the life of the implant, until the danger of disease has passed, or until it is released by cellularly-derived secretions or active cellular proteins such as a generated acid or an enzyme. Acid-labile moieties in the linkages or organosilanes bonded to the therapeutic molecules may include but are not limited to an amide linkage, an ester linkage, methylmaleamide, or a hydrazone linkage. The methylmaleamide and hydrazone linkages are stable at physiological pH, but are readily hydrolyzed in mild acid, pH 5-6. Preferably the acid-labile linkage permits release of about 50% of the therapeutic molecules in the first 60 min at a pH of between 5 and 6. Preferably the enzyme cleavable linkage permits release of about 50% of the therapeutic molecule in the first 60 minutes at a desired enzyme concentration. In a preferred embodiment the modified implant surface supports osteoblast proliferation and maturation in the presence and absence of low-level bacterial infection. A linker containing oligo(ethylene glycol) terminated by the vancomycin ligand, D-Ala-D-Ala (Ti-Si-Ala) may be made as illustrated in FIG. 7. Vancomycin has a high affinity for its ligand and in this way could be bound to the surface via formation of a complex with the ligand. In the presence of bacteria, the oligo(ethylene glycol) linker would allow the ligand-antibiotic complex to traverse the cell wall and the vancomycin would be competitively released to inhibit peptide-glycan synthesis. Other suitable antibiotic/affinity ligand complexes may also be tethered to the implant surface; combinations of antibiotics and various linkages on the implant surface may also be made.

Substrates of the present invention are biologically compatible materials and which form condensation products with organosilanes. These implant materials may include but are not limited to prosthetic, surgical, dental, and orthopedic alloys that include titanium and its alloys and is preferably Ti6A14V. Implants may also have layers that include titanium that has been sputtered or vapor deposited onto the implant. Other useful materials include but are not limited to tantalum, CoCrMo alloys, stainless steels, cobalt, chromium, aluminum, zirconium, gold, silicon and their alloys, Teflon/PTFE, polyethylene, including ultra high molecular weight polyethylene, silicone, silica, glass, and polystyrene. Ceramic materials such as zirconia may also be used as implants. Preferably the biologically compatible implant materials have surfaces that can form condensation products with organosilane. These materials may be hydrated, have one or more hydroxide groups on their surfaces, or may be treated to form such surfaces. These materials may be molded, machined or shaped for a particular implant application and maybe monolithic, a porous materials such as foams, sintered beads, woven surfaces, meshes, and other materials designed to increase surface area, porosity, fluid exchange and interconnectivity.

Therapeutic molecules of the present invention may be covalently bonded to the surface of the implant using organosilanes to form modified silylated surfaces on the implant. Organosilanes, RₙSiX₍₄₋ₙ₎, useful in the present invention include those where the hydrolyzable group X is halogen, alkoxy, acyloxy or amine and R is a nonhydrolyzable organic radical that posses a therapeutic molecule or a chemical functionality that may be use to later covalently bond to a therapeutic molecule or a linker. The organosilane forms stable condensation products with materials having oxide and or hydroxide groups on their surfaces. While not wishing to be bound by theory, one or more covalent bonds from each silicon of the organosilane forms a bond with the substrate surface as schematically illustrated in FIG. 1. A monolayer of the organosilane on the surface is preferred; however, multiplayer polysiloxanes may be formed depending upon the concentration of the organosilane used. Preferred organosilanes include but are not limited to those where R includes an amine, a carboxylic acid, epoxide, cyanide, or sulfhydryl group. Examples of such organosilanes include but are not limited to 3-aminopropyltriethoxysilane (APTS), 3-carboxypropyltriethoxysilane (CPTS), (3-glycidoxypropyl)trimethoxysilane (GPTS), and 3-mercaptopropyltrimethoxy silane (MPTS). Carboxylic acid terminated implant surfaces may also be formed by treatment of APTS surfaces with succinic anhydride. For example, APTS silylated implant substrates can be prepared by etching the substrates with phosphate fluoride and then treated with 0.2-1.0 mM 3-aminopropyltriethoxysilane (APTS) in hexane for 45-90 min to silylate the surface The implant substrates can then be sonicated in a hexane bath to remove excess reactants. The amino or carboxy substrates can provide the base for Fmoc coupling of linkers, amino acids and therapeutic molecules such as vancomycin.

The activity of cells, their secretions, or other physiological events in a mammalian patient may be used to trigger the release of therapeutic molecules, peptide, or drugs from the surface modified implants of the present invention. Non-limiting examples of such secretions or cellular events include the acid released by bacteria, metalloproteinases characteristic of cancer cells, enzymes from clots associated with bony non-unions, enzymes from clots associated with stents, and drug release predicated upon a specific event such as altered insulin level, glucose level or prostaglandin level. To be more specific, in the case of cancer cells, one novel application is to the bony tumors arising from metastases or from osteosarcomas. In this case, an implant would be modified with tethered chemotherapeutic agents that can be released upon secretion of high concentrations of metalloproteinases, enzymes that are characteristic of metastatic tumors and used for extracellular matrix degradation. This device could then serve as an active defense against re-establishment of the tumor at that site. In the case of bony non-unions, again, an implant with tethered bone-inducing factors, such as the bone morphogenetic proteins (BMP's) can be engineered to release BMP's upon dissolution of the blood clot associated with the implantation, again using enzyme activities associated with the degradation of the clot, such as the plasmin sequence. Conversely, implants could be engineered to release the growth factor during the initial stages of osteolysis to prevent implant loosening. Additionally, clot formation within a stent could be inhibited through release of thrombolytic drugs such as tissue plasminogen activator or anistreplase at the site of clot formation.

To minimize development of patient resistance to antibiotics during a course of treatment, many antibiotics such as rifampin are commonly given as a combination therapy with other drugs, such as isoniasid or ethambutol. Additional drugs can be attached by covalent bonding to a tether also chemically bonded to the implant with the antibiotic or chemotherapeutic to allow maximal efficacy in the treatment of periprosthetic infections or cancers.

Several factors have been identified in the mechanism of osseointegration and bone ingrowth that lead to long-term implant stability. Osteoblast-like cells adhere more rapidly to metallic surfaces than to standard polystyrene tissue culture surfaces and the rate of adhesion is related to the roughness of the surface (porous coating > rough > grit polished). Enhanced cell adhesion to the implant is accompanied by increased osteoblastic maturation, matrix production and mineralization. Surface chemistry plays a role in osteoblastic cell adhesion (Ti6A14V > CoCr). Cell adhesion enhancement appears to be mediated by increased expression of the matrix protein, fibronectin, and its cognate membrane receptor, α5β1 integrin. The fibronectin-integrin interaction likely serves a subcellular signaling role as well as mediating adhesion, and osteoactive factors, TGF-β1 and BMP-2, stimulate osteoblast adhesion via enhanced fibronectin and α5β1 integrin expression. Preferably the implant surfaces of the present invention incorporate such materials, morphology, and factors onto the surfaces where possible.

Vancomycin is a therapeutic molecule that may be tethered to an implantable substrate surface, for example a Ti surface, using a flexible linkage that is not cleaved and places the vancomycin within the cell (Ti-O-Si-OEG-VAN) as shown in FIG. 6. This linkage can contain vancomycin tethered to an oligo(ethylene glycol) linker that then continues into a flexible linker for attachment to the Ti surface. Vancomycin inhibits bacterial cell wall synthesis by inhibiting peptidoglycan synthesis, a process that occurs on the exterior surface of the cell membrane. Membrane-anchored vancomycin would be expected to display an increased activity against resistant organisms. Immobilized vancomycin on a flexible, non-labile hydrophobic linker, could be bactericidal without release from the Ti surface. The presence of the oligo(ethylene glycol) linker adjacent to the vancomycin can allow passage through the bacterial cell wall while still maintaining contact with the Ti surface as shown schematically in FIG. 6.

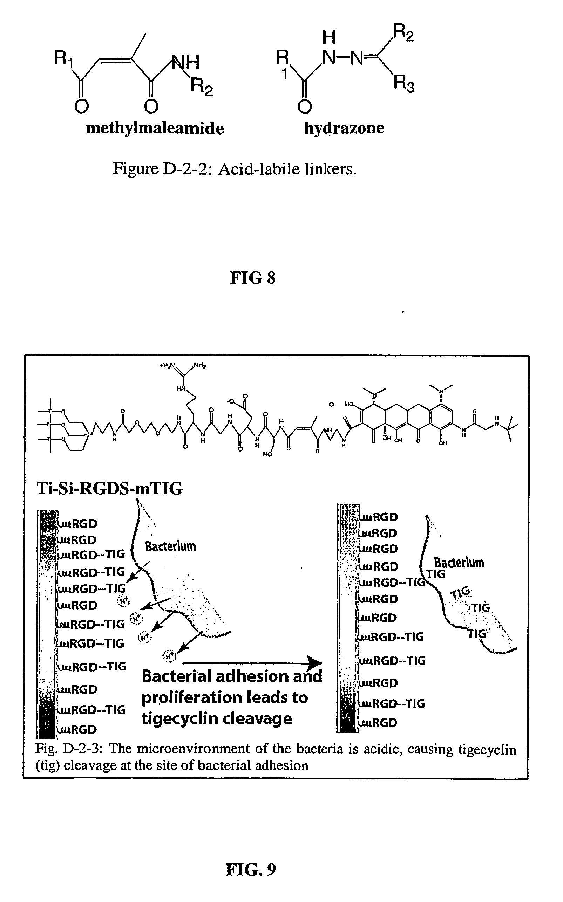

Tigecyclin is a therapeutic molecule that is an antibiotic that may be tethered to a Ti implant surface. Tigecyclin is a glycylcycline derived from minocycline with potency against tetracycline-sensitive and -resistant bacteria and thus is active against a broad spectrum of gram-positive and gram-negative bacteria. It acts by inhibition of the 30S ribosomal subunit, thereby disrupting protein synthesis. *In vitro,* tigecyclin has an [MYC90] of less than 0.25 mg/mL for most strains including *S. aureus;* for *E. faecalis* and *E. faecium,* this low MIC is coupled with an activity that was ~8-fold more active than linezolid and 32-fold more than quinupristin-dalfopristin. Using a pH-sensitive linkage, tigecyclin may be tethered to a linker like an RGDS (SEQ ID NO: 2) or RGD (SEQ ID NO: 1) peptide and the acid labile moiety methylmaleamide on an implant such as a Ti alloy surface (Ti-O-Si-RGD (SEQ ID NO: 1)-mTIG) as shown in FIG. 1. This methylmaleamide can be cleaved under mildly acidic conditions (pH 5 - 6) and tigecyclin is released into the bacterial slime where it can kill the adhered microorganisms. Cleavage of the acid-labile linkers to release the antibiotic will expose the RGD (SEQ ID NO: 1) sequence as illustrated in FIG. 9(A) and FIG. 9(B), and support osteoblast proliferation. Extracellular acid generated by bacteria can cleave the acid-labile maleamide bond, resulting in the release of tigecyclin at the bacterial cell wall. The infection is eliminated at its source in a time- and site-specific manner. The antibiotic may be active against gram-negative bacteria, active against both gram-positive and gram-negative bacteria, and preferably the antibiotic is active against gram-positive bacteria. Other therapeutic antibiotic molecules bonded to the implant surface may include but are not limited to minocyclins, Tigecyclin/GAR-396, the tetracyclin and glycylcycline antibiotics, vancomycin and its analogs, Rifampicin and its family members, Methcillin and its analogs, Gentamycin and its analogs and combinations of these.

The therapeutic molecule rapamycin is a macrocyclic triene antibiotic produced by Streptomyces hygroscopicus that binds to the cyclophilin receptor and has been shown to inhibit the proliferation of vascular smooth muscle cells. Rapamycin may be utilized in treating intimal smooth muscle cell hyperplasia, restenosis, and vascular occlusion in a mammal, particularly following either biologically or mechanically mediated vascular injury. Such injury initiates a thrombotic and inflammatory response. Cell derived growth factors such as platelet derived growth factor, basic fibroblast growth factor, epidermal growth factor, thrombin, etc., released from platelets, invading macrophages and/or leukocytes, or directly from the smooth muscle cells provoke a proliferative and migratory response in medial smooth muscle cells. Daughter cells migrate to the intimal layer of arterial smooth muscle and continue to proliferate and secrete significant amounts of extracellular matrix proteins. Rapamycin functions to inhibit smooth muscle cell proliferation and does not interfere with the re-endothelialization of the vessel walls. These secretions could be used to trigger release of rapamycin bonded to an implant surface of such as a stent to prevent or restinosis.

In addition to being an effective anti-coagulant, heparin has also been demonstrated to inhibit smooth muscle cell growth in vivo. Thus, heparin may be effectively utilized in conjunction with rapamycin as a therapeutic molecule bonded to an implant surface in the treatment of vascular disease. Essentially, the combination of rapamycin and heparin may inhibit smooth muscle cell growth via two different mechanisms in addition to the heparin acting as an anti-coagulant.

Antiinflammatory molecules may also be bonded to the implant substrate by reaction with silanized implant surfaces. Preferably the non-steroidal anti-inflammatory molecules include those which inhibit the synthesis of prostaglandins. Antiinflammatory molecules include but are not limited to acetylsalicyclic acid, indomethacin, ibuprofen, acetaminophen, apazone, celecoxib, and rofecoxib.

Oligonucleotides may also be bonded to the surface of an implant either alone or in combination with other therapeutic molecules. For example, RGD (SEQ ID NO: 1) peptide motifs may serve as part of the linker for the immobilization of therapeutic oligonucleotides to the implant. The RGD (SEQ ID NO: 1)-oligonucleotide linkage may be designed to be labile to matrix metalloproteinases released by cancer cells, thereby exposing additional RGDs (SEQ ID NO: 1) for promotion of osseointegration as the therapeutic oligonucleotide is released to the cancer cells adjacent to the implant. Peptide sequences specifically cleaved by matrix metalloproteinases can be placed between the RGD (SEQ ID NO: 1) motif and the therapeutic oligonucleotide. The effect of various amino acids in linking peptides on the release of the therapeutic oligonucleotides bonded to the implant may be determined by progressively changing the number and type of flanking amino acids at each side of the RGD (SEQ ID NO: 1) motif and anchor on the release of oligonucleotides covalently bonded to the Ti surface.

Therapeutic oligonucleotides that may be bonded to the silylated implant surface may include but are not limited to those anticancer oligonucleotides targeted against the MYC oncogene. Such therapeutic oligonucleotides may be composed of various combinations of modified bases, modified sugars, and modified internucleotide linkages. More specifically, the therapeutic oligonucleotides may be made up of various nucleic acids, including natural and modified DNA, RNA, and derivatives thereof with modified bases, sugars, or linkages, of which some non-limiting examples are PNA, LNA, and morpholino phosphorodiamidates. In one preferred embodiment, the therapeutic oligonucleotide targeted against the MYC oncogene may be comprised of peptide nucleic acid (PNA) residues, and linked to Ti-RGD (SEQ ID NO: 1) by a peptide sequence specifically cleaved by matrix metalloproteinases, and a basic peptide sequence conducive to cellular uptake.

Angiogenesis is the growth of new blood vessels in the body and is necessary for the repair or regeneration of tissue during wound healing. A lack of small blood vessel production can contribute to tissue death in cardiac muscle after a heart attack. In cancers, angiogenesis enables cancers to enlarge and spread. The walls of blood vessels are formed by vascular endothelial cells and angiogenesis can stimulate them to divide. In cancer, two proteins appear to be the most important for sustaining tumor growth vascular endothelial growth factor (VEGF) and basic fibroblast growth factor (bFGF). The binding of either VEGF or bFGF to its appropriate receptor on the cell activates a series of relay proteins that transmits a signal into the nucleus of the endothelial cells. The nuclear signal ultimately prompts a group of genes to make products needed for new endothelial cell growth. Such signals could be used to trigger the release therapeutic molecules bonded to an implant surface that could be used to alter angiogenesis of epithelial cells adjacent or attached to the implant surface.

Other therapeutic molecules and peptides which can be bonded to the linkers or organosilanes may be used to modify implant surfaces. Examples of such molecules include but are not limited to.chemotherapeutics like Taxol [CAS 33069-62-4], camptothecans; kinase inhibitors, such as erlotinib, flavopiridol, gefitinib, or imatinib; antirejection drugs that prevent restenosis like Sirolimus [CAS 53123-88-9] and Tacrolimus [ CAS 104987-11-3], cell growth factors, thrombolytic drugs, and osteoactive factors. Preferably the therapeutic molecules and peptides have moieties which can be readily coupled to the organic groups pendant from the silylated implant surface. Where such groups are unavailable, the therapeutic molecules and peptides may be chemically modified to provide a site for covalent bonding to the organosilane or linker on the surface of the implant. For example, the 17-hydroxyl of rifampin may be modified by esterification with 2-methyl-4-thioethylamido maleic acid to form a sulfhydryl group. This sulfhydryl group may be directly reacted with a silylated implant surface having epoxide groups to form a thioether. Such modified therapeutic molecules bonded to the surface of an implant may be characterized for their therapeutic activity using methods disclosed herein. Where reactive groups present on a therapeutic molecule lead to improper coupling with a silylated implant surface, protecting groups may be used to insure that only suitable groups like C-terminal carboxyl groups are available for coupling.

Therapeutic molecules, peptides, or therapeutic oligonucleotides may be covalently bonded using standard Fmoc coupling or other known methods to the organosilane modified implant surfaces having terminal amino, sulfhydryl, or carboxy groups. For example, a five- fold molar excess of Fmoc-aminoethoxyethoxyacetic acid linker (AEEA, Applied Biosystems) can be coupled using Fmoc reagents to silylated Ti-O-Si(CH₂)₃NH₂ substrates. The silylated substrates may be supported in a lab-made reaction column mounted on a Rainin PS3 peptide synthesizer, or an Applied Biosystems 430A peptide synthesizer, or an Expedite 8909 DNA/RNA/PNA synthesizer under anhydrous conditions. Initially two AEEA linkers can be added; 3-5 can be added if two linkers do not make the acid-labile linker sufficiently accessible. Following piperidine removal of the terminal Fmoc protecting group, the N-terminal amine can be reacted with five equivalents of 2-methyl maleic anhydride in dry CH₃CN plus one equivalent of dimethylaminopyridine (DMAP) for one hour. The Ti-substrates with a terminal methyl maleate can be washed with dimethylformamide, (CH₃)₂NCHO, then reacted with 10 equivalents of 1,2-diaminoethine plus two equivalents of N,N'-diisopropylcarbodiimide (DPC) in CHCl₃ under anhydrous conditions. A five-fold molar excess of vancomycin can be coupled to the resulting terminal amine using Fmoc reagents to yield the desired stable acid-labile Ti-O-Si(CH₂)₃NH-AEEA-AEEA-mVAN after a final wash with (CH₃)₂NCHO.

Using solid phase coupling, vancomycin or other therapeutic compound may be covalently bonded to an oligo(ethylene glycol)-Ti surface. For example, a five-fold molar excess of AEEA can be Fmoc-coupled by solid phase methods to Ti-amino substrates under anhydrous conditions. Initially five AEEA linkers can be added; 6-10 can be added if five linkers do not make the vancomycin sufficiently accessible. Alternatively, a five-fold excess of N-Fmoc-amide-dPEG₄, MW 478.5 available from Quanta Biodesign, Powell, OH, can be coupled to an APTS silylated Ti surface to give Ti-O-Si(CH₂)₃NH-OEG. Following piperidine removal of the terminal Fmoc protecting group, a five- fold molar excess of vancomycin can be coupled with Fmoc reagents to yield the desired (Ti-Si-OEG-VAN) Ti-O-Si(CH₂)₃NH-OEG-VAN as illustrated schematically in FIG. 6 after a final wash with (CH₃)₂NCHO.

A competitive ligand for vancomycin or other therapeutic molecule may be prepared by solid phase coupling of D-Ala-D-Ala to an oligo(ethylene glycol)-Ti surface. For example, a five- fold molar excess of aminoethoxyethoxyacetic acid t-butyl ester (AEEAtBu) can be Fmoc-coupled by solid phase methods to Ti-carboxy substrates under anhydrous conditions. Initially three AEEA linkers can be added; 4-8 can be added if five linkers do not make the noncovalently bound vancomycin sufficiently accessible. Alternatively, N-Fmoc-amide-dPEG₄, MW 478.5 available from Quanta Biodesign, Powell, OH, can be coupled to an APTS silylated Ti surface to give Ti-O-Si(CH₂)₃NH-OEG. Following piperidine removal of the terminal t-Bu protecting group, or terminal Fmoc protecting group, a five- fold molar excess of unprotected α-amino-D-Ala-D-Ala-t-butyl ester can be coupled with Fmoc reagents to yield the (Ti-Si-Ala) Ti-O-Si(CH₂)₃NH-OEG-D-Ala-D-Ala after a final wash with (CH₃)₂NCHO. The loaded implant substrates can be washed twice with PBS, then saturated with a 10- fold molar excess of 1 mM vancomycin in PBS for 30 min. to yield the desired (Ti-Si-Ala-VAN), Ti-O-Si(CH₂)₃NH-OEG-D-Ala-D-Ala*(VAN) as shown schematically in FIG. 7 followed by three washes with PBS to remove unbound vancomycin.

Chemical determination of the moieties tethered to the implant substrate surface can be determined by Matrix Assisted Laser Desorption Ionization-Time Of Flight (MALDI-TOF) mass spectroscopy. Characterization of the molecular ions resulting from the fragmentation of the modified vancomycin can be compared with values obtained from unmodified vancomycin and published literature values to confirm that linkages to the metal and modification to the vancomycin occur. Derivatized Ti disks overlaid with sinapinic acid matrix can be allowed to outgas under high vacuum in the Ciphergen sample chamber for 30 min. before analysis. Molecular ion formation can be achieved through firing of a 338 nm laser onto the surface of the Ti implant, causing bond breakage and release of the parent ion. Because of the innate charge of the molecular ions, the parent ion and any fragments can be accelerated into the mass spectrometer where the time of flight can be a function of the mass of the molecular ions. Computer assignment of the identities of molecular ions of Ti-Si-mVAN, Ti-Si-OEG-VAN, and Ti-Si-OEG-D-Ala-D-Ala can be based upon reported values for vancomycin and comparison with the pattern produced by underivatized vancomycin alone.

Detection of amount and density of acid-labile therapeutic molecules such as vancomycin bonded to a Ti implant may be determined by spectrofluorimetry and by epifluorescence microscopy. For example, tethered vancomycin can be incubated with a monoclonal antibody to vancomycin (US Biologicals, 1:100 dilution), 25°C, 60 min. in Tris-buffered saline containing 1% goat serum, followed by reaction with a sheep anti-mouse FITC-coupled secondary antibody (Molecular Probes, 1:80 dilution), 25°C, 60 min. Quantities can be estimated by comparison with known amounts of vancomycin in the fmol-nmol range. Vancomycin density on the Ti surface can be determined by epifluorescence microscopy using a TRITC-linked secondary antibody to the vancomycin antibody. Digital images can be collected using a confocal scanning laser microscope to image titanium surfaces. The confocality allows filtering out of the out-of-phase reflected light from the metal surface.

Incubation of surfaces with weak acid may be used to characterize the stability and release of tethered or complexed bioactive or therapeutic molecules bonded to an implant substrate surface. For example, Ti- vancomycin hybrids can be incubated in PBS (pH 5.0 - 8.0) for 1-180 min. The PBS pH can be adjusted using the H₃PO₄:Na₂BPO₄ ratio, while holding ionic strength constant. The most "active" pH values, which may be pH 5.0, 5.5, and 6.0, as well as physiological pH 7.4 may be evaluated in a time course where antibiotic release can be measured at 1, 2, 3, and 24 h.

The activity of the surface modified implant substrates with covalently attached, labile or complexed therapeutic molecules may be measured by incubating them in the presence of the cells that the molecules are chosen to interact with. For example, the bactericidal activity of tethered vancomycin against *S. aureus* infections may be measured. The bactericidal activity of these surfaces may be tested against an initial inoculum of 10³ to about 10⁶ *S. aureus,* a concentration that can occupy less than 1% of a sample 177 mm² modified Ti disk. Control surfaces can test the proliferation of the bacteria on the Ti alone, the effect of soluble vancomycin on bacterial propagation, the effect of the linker alone, and finally the effect of irreversibly immobilized vancomycin without OEG on bacterial proliferation. The proliferation curve of the bacteria can be determined every 60 minutes for 4 h by extraction of the DNA and measurement using PCR. Numbers of bacteria can be estimated by comparison with PCR signals using DNA isolated from known numbers of bacteria. In addition, pH measurements can be performed to determine if bulk changes in pH are occurring as a result of bacterial propagation. Finally, bacterial number can be measured by direct counting after plating serial dilutions on agar plates. Colony formation can be assessed by cell counting after photography and total numbers of colonies calculated. These values can be compared with the numbers determined by PCR. A Molecular Probes "Live/Dead BacLight" kit may also be used to visualize bacterial viability based on differential staining.

Implant substrates or portions thereof that are modified or unmodified can be sterilized by exposure to UV light and placed into the well of a 24-well tissue culture plate. 0.5 mL of DMEM containing 10% FBS can be added and medium can be inoculated with 10³- 10⁶ infectious units of *S. aureus.* The inoculum can be mixed by agitation on a rotary shaker for 5 minutes, followed by incubation at 37°C under normal tissue culture conditions, i.e., in a 5% CO₂, humidified incubator with no agitation. Bacteria proliferation can be measured as a function of time (1 - 24 h).

Isolation of DNA from adherent and non-adherent bacteria. Non-adherent bacteria can be gathered in the supernatant, pelleted, and resuspended in 100 µL PBS. Adherent bacteria on the surface can be bathed in 100 µL PBS. Cell lysis can be achieved by four freeze/thaw cycles, followed by centrifugation. The supernatants can be used for determination of bacterial number by PCR.

PCR may be used to determine bacterial number. For example, 10 -20 µL of the above DNA solutions can be used in a 100 µL reaction volume using a Tris-KCI-MgCl₂ buffer and AmpliTaq, as described in the paper. Primers are directed at the 16S rRNA (Forward: CGGCAGGCCTAACACATGCAAGTCG. Reverse:
GGTTGCGGCCGTACTCCCCAGG) to yield an 881 bp product, after 30 -35 cycles.
Detection can be by ethidium bromide or SybrGreen II staining after fractionation by agarose gel electrophoresis.

Determination of *S. aureus* numbers by dilution. Supernatant can be removed from cultures containing *S. aureus* as above and the chemically modified surfaces can be washed with 2 mL of DMEM and pooled with the supernatant from the same culture. Following agitation, this supernatant can be plated on LB agar plates at dilutions of 0, 10², 10⁴, 10⁶ for determination of bacterial numbers. Additional dilutions can be used, as appropriate, to yield a countable number of colonies on the plate. Surfaces can be photographed and cell numbers determined by direct cell counting. Determinations can be performed by two or more independent investigators.

Visualization of adherent and floating viable and non-viable bacteria. Using the Live/Dead BacLight system (Molecular Probes), live and dead bacteria can be stained with SYTO9 + propidium iodide. Live cells can fluoresce green, whereas cells with compromised membranes can fluoresce red. Digital images can be recorded of surface adhered and floating bacteria and ratios of live:dead bacteria determined in random fields using double immunofluorescence.

The activity of various therapeutic molecules and their biocompatibility may be determined by measuring the proliferation and maturation of target cell on modified implant surfaces. For example, the bactericidal activity and biocompatibility of tethered vancomycin of the present invention can be determined in the presence of cells. The effect on the proliferation and maturation of chondrocytes and of osteoblasts, the cell types important for fracture healing can be measured. Importantly, the surface as designed, should be stable to both chondrogenic and osteoblastic cell proliferation, i.e., preferably release antibiotic, as appropriate, in the presence of bacteria. Using two concentrations of bacteria that were killed when incubated with the active surface, the effects of a culture with ATDC5 pre-chondrogenic and MC3T3-E1 osteoblast-like cells can be determined. These cells can be plated at a density of 1x10⁴ cells/1.77 cm² implant substrate at the same time as inoculation of bacteria.

For the acid-labile linkage, acidic solutions can cause significant, and time-dependent release of a therapeutic tethered molecule like vancomycin. In the neutral pH range, there may be extremely slow or minimal release of the therapeutic molecule from the implant. For the covalent linkages, such as OEG-VAN, and complexed bioactive molecule such as Ala surfaces, the linkages should be pH stable. Should the acid-labile linkage cause an immediate release of antibiotic, the linkage may be modified so as to decrease its acid lability. This may be done by decreasing the electron density at the cleavage site making it less likely to be protonated. Preferably the site of modification of the therapeutic molecule is such that the activity of the molecule is not reduced. The active sites of the molecules would be known to those skilled in the art.

The amount and number density of therapeutic molecules tethered to the implant surface that are required to produce an effect on cells near the substrate can be determined by measurement of the numbers of adherent and planktonic bacteria or other cells via PCR and colony formation assays. The live to dead ratio of cells on the surface of surface modified substrates and in solution using dyes specific for living or dead cells such as bacteria may also be used. Should surface bound therapeutic molecules be insufficient to interact with cells and cell colonies near the implant surface, multivalent linkages may be used to increase their surface concentration.

Embodiments of the present invention include surfaces with important clinical applications. The first surfaces include therapeutic molecules tethered or associated with an implant surface through a ligand or linker connecting the therapeutic molecules and implant surface. Additional modifications are possible to further develop these surfaces, as are the addition of other site-directed delivery systems.

Testing of the covalently modified or derivatized surfaces applied to substrates such as an intramedullary nail in an animal models can be used to establish whether the derivatized implants maintain their therapeutic (chemotherapeutic, anti-inflammatory, proliferative, or bactericidal) activity *in vivo* and act to heal fractures or perform sensing functions. Substrates, such as but not limited to a layer of tethered therapeutic molecules on an implantable substrate may be evaluated using animal testing. Without limitation and for illustrative purposes only, suitable therapeutic molecules may include an antibiotic, and the implantable substrate an intramedullary nail.

Quantitative microCT can be used to assess the structural properties of the developing fracture callus and the formation of trabeculae in the healing cancellous bone. Micro-tomographic imaging values are in excellent agreement with the indices assessed from conventional histomorphometry and are an excellent predictor of bone quality and strength. In vivo microCT analysis may be used to dynamically monitor the healing of the bone, its structure, and its mechanical strength, over the course of animal experiments. An infected fracture cannot heal properly and the microCT can be used to provide an indication of an establishing infection and the performance of a surface modified substrate.

Covalently modified intramedullary nails can be implanted into the femurs of Wistar Rats and the femurs fractured using an Einhorn protocol. After confirmation of fracture by radiology, an infectious agent such as *S. aureus* can be injected into the site of the fracture. To prevent sepsis, the concentration of *S. aureus* can be kept very low (1-50 cfu). Ten rats can comprise a sample group, with a treated nail inserted on one side and an untreated nail inserted on the contralateral control side. Using this general testing methodology, it is also possible to evaluate the effects of an implant surface modified by covalent attachment of linkers and therapeutic molecules. The fracture sites can be monitored at 7, 14 and 21 days. Animals can be sacrificed at these time points and fracture healing assessed by conventional histology and morphometry. Evaluation of the systemic infection and serum antibiotic levels may be determined at these time periods. MicroCT can also be used to monitor fracture healing at 0, 7, 14 and 21 days, as well as to obtain information on the mechanical strength of the bone. At the termination of the experiment, bone can be X-rayed, harvested and its mechanical properties measured.

Skeletally mature, male, Wistar rats, weighing 300 - 500 grams, can be anesthetized by intraperitoneal injection of ketamine (20-60 mg/kg) and xylazine (2.5 mg/kg) anesthesia. Hind limbs can then be shaved, washed with Betadine (Povidine-Iodine), and under aseptic conditions, a medial parapatellar arthrotomy made and the patella dislocated laterally. The intramedullary canal can be entered through the intercondylar notch and reamed with an 18-gauge needle. Modified or unmodified intramedullary nails can be prepared from 1.14 x 26 mm Kirschner wire (Smith and Nephew Richard, Inc., Memphis, TN, USA). The nail can be inserted into the canal, the patella relocated, soft tissues closed with 4.0 nylon sutures, and the skin closed with staples. This stabilized femur can be placed in a three-point bending device and the femur fractured by the force of a 500-gram weight dropped 35 cm. Bilateral fractures can be made in all animals. X-ray analysis can be used to confirm the position and orientation of each fracture. Fractures outside the central 8mm of diaphysis, and oblique or spiral fractures, can be excluded from the study. After the animals are killed, the femora can be disarticulated and radiographs made for assessment of fracture-healing. Each fracture specimen can be reviewed and classified for callus maturity according to the classification wherein stage 1 indicates nonunion; stage 2, possible union; and stage 3, radiographic union.

After confirmation of the fracture by microCT, an aliquot containing about 10⁴ to about 10⁶ cfu of *S. aureus* in 100 µL can be injected into the medullary cavity.

All nails can be fitted snugly and no other fixation used postoperatively. Rats can be allowed unrestricted ambulation in their cages after recovery from anesthesia, and can be observed daily for activity and weight bearing as indicators of adequate fracture immobilization and freedom from pain. All femora can be imaged again at the time of sacrifice, with the contralateral femora serving as control. At 7, 14, and 21 days, i.e., the time of sacrifice, blood samples can be collected for determination of blood levels of bacteria and antibiotics. Harvested tissues can also be assessed for bacterial infection. At 7, 14 and 21 days, aliquots of blood taken from the test mammals can be evaluated for antibiotic load and the presence of *S.aureus.*

Femora from five animals from each sample can be utilized for histological and morphometric analysis, as known to those skilled in the art. Immediately after animal sacrifice, fracture calluses can be removed, fixed for two to three days in 10 percent neutral buffered formalin followed by two days in Bouin's solution and then decalcified in a 10 percent acetic acid, 0.85 percent NaCl, and 10 percent formalin solution. Since the nail cannot be integrated, it can also be retrieved aseptically for testing of antibiotic load, infection status and surface characteristics. Specimens can be embedded in paraffin, sectioned longitudinally, and stained with hematoxylin and eosin or Masson's trichrome. The progression of fracture-healing in each specimen can be measured with use of a scale that assigns a grade based on the relative percentages of fibrous tissue, cartilage, woven bone, and mature bone in the callus. Grade I indicates fibrous tissue; grade 2, predominantly fibrous tissue with some cartilage; grade 3, equal amounts of fibrous tissue and cartilage; grade 4, all cartilage; grade 5, predominantly cartilage with some woven bone; grade 6, equal amounts of cartilage and woven bone; grade 7, predominantly woven bone with some cartilage; grade 8, entirely woven bone; grade 9, woven bone and some mature bone; and grade 10, lamellar (mature) bone. Four slides can be examined for each fracture.

Histomorphometric evaluation of trabecular structure and chondrocyte characteristics can be carried out with an Osteo Metric's image-analysis software system (Atlanta, Georgia). Measurements of trabecular structure and of the size and number(cell density) of hypertrophic chondrocytes can be made in the interface region (the region beneath the subperiosteal new bone formed by intramembranous ossification and the ends of the fractured femur). Measurements of the hypertrophic chondrocytes can be made in the cartilage within about 258.5 micrometers of this interface. Parameters that can be evaluated include cell size (in square millimeters) and cell density (in cells per square millimeter). Evaluation of endochondral bone included (1) the area occupied by trabeculae composed of spicules of calcified cartilage covered by osteoid and newly formed bone, (2) the thickness of these trabeculae, and (3) the percentages of bone and calcified cartilage in these trabeculae.

For the microCT imaging, the unprocessed fractured bone of the living rats can be evaluated at 0, 7, 14, and 21 days using a compact fan-beam-type tomograph (Scanco Medical AG, Bassersdorf, Switzerland). A typical examination can consist of a scout view, selection of the examination volume, automatic positioning, measurement, offline reconstruction, and evaluation. For each sample, a total of 286 micro-tomographic slices with a slice increment of 14 µm can be acquired covering 4 mm of the total length of the fractured bone. The total examination time per rat limb can be approximately 30 min for a voxel size of 14 cm³. For the subsequent analysis, a subvolume of the originally measured data can be selected. This selected volume of interest (VOI) can be located in the center of the fracture site. The size of the VOI can be set at 4 mm³ (286 x 286 x 286 voxels). In the next step, bone tissue can be separated from marrow using a thresholding procedure. All samples can be binarized using the same parameters for the filter width, the filter support, and the threshold (about 10.2% of maximal possible gray value). For the 3D visualization an extended marching cubes algorithm may be used. At the completion of the study, and after animal sacrifice, the healing bone can be evaluated by the conventional techniques.

The efficacy of a tethered bioactive molecule such as vancomycin covalently bonded on an implant to minimize biofilm formation may be determined by recovery of the implant at the end of the animal study and its incubation in an LB broth. Serial dilution techniques may be used to determine the number of bacteria that remain on the implant surface. The contralateral untreated nail can be used as a control. Because the infection is systemic, both nails should be equally exposed to the presence of bacteria.

The stability of a tethered therapeutic molecule such as vancomycin covalently bonded to an implant surface to the *in vivo* conditions can be determined after retrieval of the surface-modified implant nails. The retrieved, derivatized or modified implant can be washed extensively and then for example reacted with the antibody to vancomycin. Reaction with a FITC-labeled secondary antibody and imaging by confocal laser scanning microcopy can give a rough estimate of the density of the antibody remaining after the *in vivo* incubation. As controls, modified but unimplanted substrate nails can also be visualized to determine vancomycin surface density.

Specimens can be kept moist, wrapped in saline saturated gauze, and stored in air tight containers at 4°C until day of testing. On the day of testing, femora can be kept moist and brought up to room temperature, mounted in bone cement to leave a 15 mm length of diaphysis, and subjected to mechanical testing.

Each femur may be subjected to torsion at 15°/min until failure using a jig that ensures proper alignment and that is designed to convert translational displacement to angular displacement using an Instron hydraulic materials testing machine. Load-displacement curves may be continuously recorded on a plotter, digitized using a flat-bed scanner, and analyzed using Scion Image, beta version 3b (Scion Corporation, Frederick, Md). Ultimate torque (N-m), stiffness (N-m/deg) (slope of load-deformation curve measured within proportional range), angle of deformation to failure (deg), and energy absorption to failure (N-m x deg) (area under the load-deformation curve) can be calculated. Gross failure patterns can be detected visually and can be classified as (I) failure through the fracture site and adjacent host bone, or (II) failure independent of the fracture (i.e. through host bone only).

All data can be analyzed by one-way and two-way ANOVAs to test statistical significance (p<0.05) between groups and time points. Multiple, planned comparisons between groups and time points can be performed using orthogonal contrasts (SYSTAT, Systat, Inc., Evanston, IL).

Correlations can be performed to study the interdependence/association between temporal changes in mechanical properties of the long bones and histomorphometric parameters at the fracture site. Pearson product-moment correlation coefficients, r, can be determined for each mechanical property versus each histomorphometric parameter over time. The average of each variable per experimental group per time point can be used. The null hypothesis, no correlation, can be rejected for a significance level of 5%.

For implants modified with therapeutic molecules to prevent bacterial infections, restenosis in implanted stents, tumor recurrence in a previously treated tumor, fouling of a sensor, or other cellular conditions, the outcome of animal study tests would be surfaces free of or having substantially reduced numbers of the undesirable cells. In the case of an implant to repair fractured bone or dentin, the outcome would be healing of the fracture without the presence of undesirable cells on the implant surface. In particular for intramedullary implants modified with antibiotics, the outcome should be infection-free, fracture healing, normal bone structure as assessed by microCT and histology, and biocompatibility as assessed by histology.

The first measure of the outcome can be in the infection-free healing of the fracture. The bacteria injected into the site of fracture will disrupt normal bone healing and may be used to monitor whether particular linkers and therapeutic molecules covalently bonded to the implant surface are effective. If a significant infection is present in the bony cavity, healing can be retarded and non- union can be the likely result. With respect to infection, therefore, the assessment of the degree of bone healing relative to that in bacteria-free controls is a measure of the effectiveness of a surface modification or derivatization. For example, the test result for the presence of an *S. aureus* infection directly upon harvest of the bone can be used to directly measure if the presence of bacteria affects the bone healing as well as if the surface modification is successful in eliminating infection by day 21. Bone structure can be assessed during healing by use of microCT as well as histology. Histological evaluations can occur on a subset of the animals throughout the healing period as well as on the remaining animals at the end of the study. The histology data can be correlated with the microCT data to assess the relative differences in trabecular structure, callus formation, mechanical strength, and bacterial load as a function of the antibiotic and the linkage covalently bonded to the implant surface. Specifically, for fractures through the aid of the microCT, the tightness of the bone interface with the nail may be determined. In cases of infection and fracture a modification on the implant surface that is not effective is expect to show an increasing space around the nail, poor healing, and soft tissue damage. In the case of an implanted sensor, the sensitivity of the sensor over the course of the animal study wherein the animal is injected with a trial of an agent may be used to assess the activity and function of a modified implanted senor surface with linkers and therapeutic molecules designed to eliminate fouling by undesirable cell growth on the sensor. Embodiments of the present invention may be further understood by reference to the following non-limiting examples.

### EXAMPLE 1

This example illustrates that implants may be derivatized by covalently bonding bioactive peptides to their surfaces.

Aminopropyl triethoxy silane (APTS) linkers can be used that allow reactions of peptides or molecules containing peptide-like moieties. Specifically, RGD (SEQ ID NO: 1) peptides were covalently bonded to a silicon wafer using APTS as a derivatizing agent. The presence of RGD (SEQ ID NO: 1) on the surface was determined by time-of-flight secondary ion mass spectrometry and surface roughness was measured by AFM. The APTS alone caused a larger increase in roughness than reaction with APTS-linked RGD (SEQ ID NO: 1), probably the result of multiple layers of APTS. Inclusion of (CH₃)₂NCHO washes and sonication after the silanization step ensures that only covalently bonded ATPS organosilane remain on the surface, with the unbound APTS removed. This experiment demonstrates that RGD (SEQ ID NO: 1) is directly linked to the silicon surface via the APTS linker.

Osteoblasts were bound to the modified surface. Spinning disc technology may be used to measure cell adhesive strength as a function of the applied centripetal force. Adhesive strength of the osteoblasts was measured by calculating the shear stress on the cells (speed of the spinning disc as a function of distance from the center of the disc). Covalently-bound RGD (SEQ ID NO: 1) increased adhesive strength significantly over physisorbed, RGD (SEQ ID NO: 1), APTS-modified silicon and silicon alone. Osteoblasts, plated on these RDG surfaces showed a well-spread morphology, characterized by large, patchy focal adhesion contacts and increased integrin expression; osteoblasts plated on control surfaces were significantly less well-spread. Furthermore, inhibition of osteoblastic attachment by integrin antibodies inhibited cellular attachment.

Without wishing to be bound by theory, it may be that adhesion of osteoblasts to an RGD (SEQ ID NO: 1)-engineered surface stimulates their maturation and mineralization. As assessed by RT-PCR, expression of phenotypic markers of osteoblast maturation was increased in cells plated on the modified surfaces. Furthermore, alkaline phosphatase and alizarin staining was also increased. FTIR analysis showed that the deposited mineral was biological apatite.

### EXAMPLE 2

This example shows how a therapeutic molecule that is an antibiotic may be immobilized by covalent bonding to an implant surface. An antibiotic can be immobilized on a Ti surface and that the tethered antibiotic maintains its antibacterial activity. Vancomycin can be used as a representative antibiotic.

For this purpose, a 4.37 g sample of 350 mesh Ti6A14V particles were cleaned with 5 mL of 50% MeOH/50% conc. HCl for 20 min. at room temperature, with vortexing for 20 sec. at 0, 5, 12, 15, and 20 min. The particles were washed twice with 5 mL of double-deionized water, then four times with 5 mL of anhydrous dimethylformamide [(CH₃)₂NCHO]. After removing remaining (CH₃)₂NCHO supernatant, the particles were dried overnight under vacuum in the entry chamber of a Vacuum Atmosphere MO-20M glove box. Next morning, the particles were taken into the argon atmosphere chamber of the glove box and washed twice with 10 mL of anhydrous toluene, resuspending them with a stainless steel spatula. Then the particles were modified with 5 mL of 5% (v/v) APTS in anhydrous toluene for 60 min., stirring the particles with a stainless steel spatula at 0, 10, 30, and 60 min. The particles were then washed twice with 10 mL of anhydrous (CH₃)₂NCHO, resuspending them with a stainless steel spatula. The supernatant was removed and the particles were dried under vacuum overnight in the entry chamber of the glove box.

Next morning, the particles were removed from the entry chamber of the glove box and a 0.1168 g sample was assayed for amine content. The sample was resuspended in 1 mL of 0.35 mM SnCl₂, 0.1 M Na citrate, pH 5, then mixed with 1 mL of 4% (w/v) ninhydrin in EtOH. The suspension was immersed in a boiling water bath for 15 min., cooled for 2 min., then diluted with 5 mL of 60% EtOH/40% H₂O. One mL of the 7 mL supernatant was diluted with 9 mL of 60% EtOH/40% H₂O. The 10-fold dilution yielded A₅₇₀=0.324. Using ε₅₇₀=1.5x10⁴/M cm, calculated = 21.6 µM, which corresponded to aminopropyl derivatization of 13.0 µmol/g for the modified Ti6A14V, lot 030712-01. The aminopropyl-modified particles of Ti6A14V will provide the support for Fmoc coupling of linkers, unusual amino acids, and vancomycin.

A Glen Research solid phase synthesis column was charged with 1.29 g of aminopropyl-Ti6A14V, lot 030712-01, and inserted into position 2 of a Rainin PS3 peptide synthesizer as shown in FIG. 1. The support was washed twice with 5 mL of anhydrous (CH₃)₂NCHO, then automatically coupled with a four-fold molar excess of Fmoc-aminoethoxyethoxyacetic acid linker activated with a four-fold molar excess of N-[(dimethylamino)-1-H-1,2,3-triazolo[4,5-b]pyridine-1-ylmethylene]-N-methylmethanaminium hexafluorophosphate N-oxide, followed by automatic Fmoc deprotection with 20% piperdine/80% (CH₃)₂NCHO. The deprotection solution and wash were collected for ultraviolet absorbance spectroscopy. A second AEEA linker was coupled, and the second Fmoc deprotection solution and wash were collected for analysis. Similarly, a four-fold excess of vancomycin was coupled automatically by the same protocol.

After the third coupling, the column was washed twice with 5 mL of anhydrous (CH₃)₂NCHO, then dried under vacuum overnight. Absorbance spectra of the deprotection solutions revealed 301 nm peaks characteristic of the 9-piperidino-dibenzofulvene product of Fmoc deprotection. Using ε₃₀₁=7.78x10³/M cm, it was calculated that quantitative addition of the two AEEA linkers to the aminopropyl-Ti6A14V support occurred. The VAN-AEEA-AEEA-NH-Pr-Ti6A14V preparation (Van-Ti), lot 030718-01, can be analyzed by MALDI-TOF mass spectroscopy, then tested for its ability to bind peptidoglycan and to inhibit growth of *S. aureus.*

Chemical determination of the moieties tethered to the Ti particles was analyzed by Matrix Assisted Laser Desorption Ionization-Time of Flight (MALDI-TOF) mass spectroscopy. Derivatized Ti particles overlaid with a 2,5-dihydroxy-benzoic acid matrix were allowed to outgas under high vacuum in the Ciphergen sample chamber for 30 min. before analysis. Molecular ion formation was achieved through firing of a 338 nm laser onto the surface of the Ti, inducing bond breakage and release of a spectrum of product ions, which were accelerated into the mass spectrometer by a strong magnetic field. The mass spectrum observed is shown in FIG. 2. The time of flight in this situation is a function of the mass of the molecular ions. In this first attempt to sputter VAN fragments from the Ti surface, many small peaks appeared. Assignment of the identities of molecular ions of the putative VAN-AEEA-AEEA-NH-Pr-Ti6A14V were attempted based on the known masses of vancomycin, 1449 Da, and the AEEA linker, 163 Da. Reasoning from the structure, the 1537 Da peak might be VAN-NH-EtOEtOH, the 1472 Da peak might be a Na⁺VAN, and the 1402 Da Peak might be decarboxylated VAN.

Lys-D-[¹⁴C]Ala-D-[¹⁴C]Ala soluble peptidoglycan binding to VAN-Ti. An empty polypropylene vial and 100 mg samples of Ti6A14V, aminopropyl-Ti6A14V, lot 030712-01, and VAN-AEEA-AEEA-NH-Pr-Ti6A14V, lot 030718-01, were resuspended in 1 mL of PBS, and rocked for 1 hr at room temperature. The supernatants were removed and replaced with 2.5 nCi of Lys-D-[¹⁴C]Ala-D-[¹⁴C]Ala soluble peptidoglycan, and then rocked for 2 hr. The supernatants were removed and counted in 10 mL of Aquasol II (PerkinElmer Life Sciences) in a Beckman LS 6000SC scintillation spectrometer. The VAN-Ti preparation bound more of the labeled peptidoglycan (50-60%) than aminopropyl-Ti6A14V or unmodified Ti6A14V (15%). To reduce the potential for nonspecific binding, the experiment was repeated, replacing the PBS in the washing step with 0.1% (w/v) bovine serum albumin in TBST. The samples were rocked at room temperature for 4 hours and overnight at 4°C. After three washes with PBS to remove unbound label, the supernatants were removed and counted. Binding was reduced in each sample subsequent to the albumin blocking step. The vancomycin-containing derivative bound 25% of the label, whereas the other two derivatives bound 5% or less.

### EXAMPLE 3

This example illustrates the bactericidal activity of a therapeutic antibiotic molecule vancomycin bonded to an implant surface, VAN-Ti, against *S. aureus* infections.

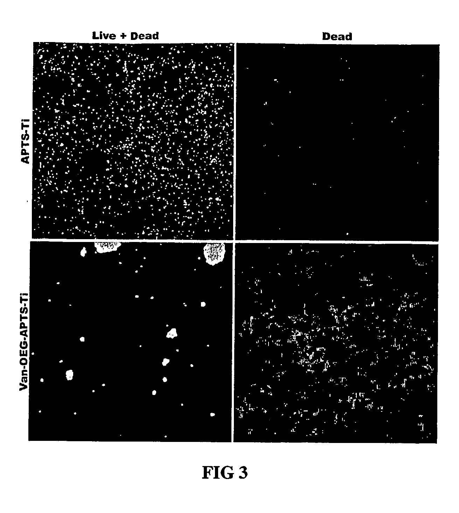

To assess the bactericidal activity of the Ti-grafted antibiotic, the following experiment was performed. Ten µL of an overnight culture of *S. aureus* was inoculated into 2 mL of LB/1% dextrose, incubated for 2 h at 37°C with vigorous aeration, pelleted, resuspended in 1 mL and 10 µL of that culture used to inoculate wells containing 200 µL PBS/1% dextrose and APTS-derivatized Ti, VAN-Ti, or PBS alone; modified Ti samples had been subjected to three PBS washes prior to use. Samples were incubated for 1 h at 37°C, washed with PBS, and stained with the Live/Dead® BacLight^{™} Bacterial Viability Kit (Molecular Probes). Live and dead bacteria were visualized by confocal microscopy. The results of representative fields resulting from this treatment are presented in FIG. 3. *S. aureus* incubated on Ti that has been derivatized with the linker APTS are shown in the top row (A) and (B). Most bacteria stain green (light gray), with only dead cells exhibiting a red fluorescence (light gray). Many live bacteria are evident, with a small amount of red staining as would be expected in any culture of bacteria. The bottom row, (C) and (D) shows *S. aureus* that were incubated on the vancomycin-tethered surface (VAN-OEG-APTS-Ti) Note the reduction in the lawn of live bacteria, predominantly black (C) and a concomitant increase in the numbers of dead bacteria by red fluorescence (light gray (D)). The results clearly indicate that the vancomycin derivatized Ti surface retains its antibiotic activity and kills the adherent *S. aureus.* The results show that an active antibacterial surface was created on an implant and that such implants are useful in treatment or prophylaxis of periprosthetic infection.

### EXAMPLE 4

This prophetic example demonstrates preparation and characterization of a surface with therapeutic molecules covalently bonded to an implant surface that enhances bone cell attachment and thereby facilitates tissue-implant integration, while remaining a long-term reservoir of antibiotics to eradicate any infections that arise from hematogenous sources.

The implant can include covalently linked RGD (SEQ ID NO: 1)-containing peptides to a Ti, creating a peptide-Ti hybrid. This can provide a mixed surface as some of the RGD (SEQ ID NO: 1) motifs will serve as part of the linker for the immobilization of the therapeutic molecule tigecyclin. The RGD (SEQ ID NO: 1)-tigecyclin linkage will be labile, thereby exposing additional RGDs (SEQ ID NO: 1) for promotion of osseointegration as the antibiotic is released.

For enhanced osseointegration, the RGD (SEQ ID NO: 1) peptide motif can be used; it is one of the most common cell binding ligands; and it is contained within the major bone proteins (e.g., collagen type I, osteopontin and bone sialoprotein). In addition to providing an immediate connection between the cell and the surface, the interaction between RGD (SEQ ID NO: 1)-containing peptides and integrin receptors play a significant role in the regulation of osteoblast function and bone development. In bone, these markers include elevation of alkaline phosphatase activity, increased osteocalcin, osteopontin and type I collagen expression, and evidence of mineral deposition. The binding activity of the RGD (SEQ ID NO: 1) motif is modulated by flanking amino sequences. For example, the loop region of the attachment portion of fibronectin includes both RGD (SEQ ID NO: 1) as well as flanking amino acids such as but not limited to (PGVDYTITVYAVTGRGDSPASSKPVSINYR) (SEQ ID NO: 6). The effect of such flanking amino acid sequences may be determined by progressively increasing the number and type of flanking amino acids at each side of the RGD (SEQ ID NO: 1) motif and anchor on the modified peptides covalently bonded to the Ti disc.

Cells will be plated on RGD (SEQ ID NO: 1)-modified surfaces and cell binding and differentiation will be evaluated. Cell binding will be evaluated using both cell attachment assays and the spinning disc methodology, while differentiation will be evaluated using RT-PCR evaluation of osteoblast phenotypic markers, as well as measurement of alkaline phosphatase activity and mineral deposition. Controls for the study will include motifs such as RGE, RGES and scrambled peptides, as well as unmodified Ti and linker-modified Ti.

The broad spectrum, new generation antibiotic tigecyclin will be tethered to the metal on a background of this adhesion peptide to yield a surface that is both osteogenic and bactericidal. Tigecyclin may be linked to Ti-RGD (SEQ ID NO: 1), as illustrated in FIG. 9 (A), by acid-labile maleamide or hydrazone linkages as shown in FIG. 8. Both methylmaleamide and hydrazone linkages display stability at physiological pH, but are readily hydrolyzed in mild acid, pH 5-6. In the protected environment surrounding a Ti implant, bacterial proliferation leads to areas of lowered pH (in solution, this drop is >4 pH units). These linkers will be cleaved under mildly acidic conditions (pH 5.0 - 6.0), releasing tigecyclin into the bacterial slime where it will kill the adherent microorganisms. For tigecyclin to be active, it must be released from the Ti surface and taken up by bacteria. Release of tigecyclin from the Ti surface will be triggered by the acidic bacterial microenvironment. The released antibiotic will then target these bacteria. Accordingly, the infection is eliminated at its source in a time- and site-specific manner. Cleavage of the acid-labile linkers to release the antibiotic will expose the RGD (SEQ ID NO: 1) motif as illustrated FIG. 9(B).

The modified Ti surfaces can be evaluated for antibiotic stability and/or release kinetics under acidic conditions and for bactericidal activity against adherent *S. Aureus.* The modified surface may also be characterized to determine if the modified surface supports osteoblast proliferation and maturation both in the presence and absence of a low-level bacterial infection.

Ti alloy (Ti6A14V) will be purchased from Stryker Howmedica Osteonics (Allendale, NJ) as highly polished disks identical to those used in surgical practice. Surfaces will be cleaned with HCl/MeOH prior to use and will be evaluated for surface smoothness by SEM.

Modification of the Ti surface. RGD (SEQ ID NO: 1) peptides will be covalently attached to an activated, smooth Ti surface using silane chemistry. Briefly, discs will be washed with anhydrous toluene, then treated with 1.0-5.0 mM 3-aminopropyltriethoxysilane (APTS) in toluene for 45-90 min. The discs will then be sonicated in toluene followed by dimethylformamide, (CH₃)₂NCHO, to remove excess reactants. The amino discs will provide the base for Fmoc coupling of linkers, unusual amino acids, and tigecyclin.

Solid phase coupling of acid-labile Tigecyclin to the Ti surface. A five-fold molar excess of Fmoc-aminoethoxyethoxyacetic acid linker (AEEA, Applied Biosystems) will be coupled using Fmoc reagents to Ti-amino discs supported in a lab-made reaction column mounted on a Rainin PS3 peptide synthesizer under anhydrous conditions. The RGD (SEQ ID NO: 1) or RGDS (SEQ ID NO: 2) sequence, or more extensive sequences, or control sequences, will be assembled from the N-terminus of the linker by normal Fmoc coupling. The acid-labile tigecyclin derivatives will be added at only 0.5 equivalents in order to assure that at least 50% of the RGD (SEQ ID NO: 1) or RGDS (SEQ ID NO: 2) peptides are free from the outset, as illustrated in FIG. 10 (A).

Following piperidine removal of the last amino acid Fmoc protecting group, the N-terminal amine will be reacted with 0.5 equivalents of 2-methyl maleic anhydride in anhydrous (CH₃)₂NCHO plus one equivalent of dimethylaminopyridine (DMAP) for one hour. The excess unreacted N-termini will then be capped with acetic anhydride. The Ti-discs with a terminal methyl maleate will be washed with (CH₃)₂NCHO, then reacted with one equivalent of 1,2-diaminoethane plus two equivalents of N,N'-diisopropylcarbodiimide (DPC) in (CH₃)₂NCHO under anhydrous conditions for 2 hr, followed by 2 washes with (CH₃)₂NCHO. Two equivalents of tigecyclin in the carboxylate form in (CH₃)₂NCHO will be coupled to the resulting terminal amine using Fmoc activator HATU to yield the desired acid-labile Ti-Si-RGDS (SEQ ID NO: 2)-mTIG after a final wash with (CH₃)₂NCHO.

Following piperidine removal of the last amino acid Fmoc protecting group, the N-terminal amine will be reacted with 0.5 equivalents of succinic anhydride in anhydrous (CH₃)₂NCHO plus one equivalent of dimethylaminopyridine (DMAP) for one hour. The excess unreacted N-termini will then be capped with acetic anhydride. The Ti-discs with a succinate will be washed with (CH₃)₂NCHO, then reacted with one equivalent of t-butyl carbazate (Boc-NENH₂) plus two equivalents of N,N'-diisopropylcarbodiimide (DPC) in (CH₃)₂NCHO under anhydrous conditions for 2 hr, followed by 2 washes with (CH₃)₂NCHO. The Boc protecting group will be decomposed with 20% CF₃CO₂H in (CH₃)₂NCHO. The resulting hydrazide will then be coupled with two equivalents of tigecyclin in the ethyl ketone form in (CH₃)₂NCHO for 24 hr to yield the desired acid-labile Ti-Si-RGDS (SEQ ID NO: 2)-hTIG after a final wash with (CH₃)₂NCHO.

MALDI-TOF Mass Spectroscopy. Chemical determination of the moieties tethered to the Ti surface will be determined by Matrix Assisted Laser Desorption Ionization-Time Of Flight (MALDI-TOF) mass spectroscopy of products removed from the discs with aqueous 0.1% CF₃CO₂H, pH 2. Characterization of the molecular ions resulting from the fragmentation of the modified Tigecyclin will be compared with values obtained from unmodified Tigecyclin and published literature values to confirm that linkages to the metal and modification to the Tigecyclin occur as designed. To characterize peptides remaining on the acid-washed Ti discs, they will be overlaid with a sinapinic acid matrix and allowed to outgas under high vacuum in the Ciphergen sample chamber for 30 min. before analysis. Molecular ion formation will be achieved through firing of a 338 nm laser onto the surface of the Ti, causing bond breakage and release of the parent ion. Because of the innate charge of the molecular ions, the parent ion and any fragments will be accelerated into the mass spectrometer where the time of flight will be a function of the mass of the molecular ions. Computer assignment of the identities of molecular ions of Tigecyclin and RGDS (SEQ ID NO: 2) peptides will be based upon reported values for Tigecyclin and comparison with the pattern produced by underivatized Tigecyclin alone.

The surface of the modified Ti will be characterized using atomic force microscopy (AFM) and scanning electron microscopy (SEM). AFM imaging will be performed using a Dimension Bioscope atomic force microscope under fluid conditions. Topographic images will be acquired in a tapping mode using silicon tips on integral cantilevers with a nominal spring constant of 20-100 N/m. Images will be obtained from at least two different samples prepared on different days and at least three macroscopically separate areas on each sample. Scan areas will range from 10 nm x 10 nm to 10 mm x 10 mm. SEM images will be acquired with a Jeol 6300FV equipped with a field emission gun (FEG). Because of the FEG, outstanding resolution can be achieved at accelerating voltages as low as 0.5 keV which allows for imaging non-conducting materials such as cells without the need for coating. The point to point resolution at 1 keV is 7 nm and decreases to 1.5 nm at 30 keV. The thickness and stoichiometry of the Ti-peptide hybrids will be determined with Rutherford backscattering spectrometry. A 5SDH accelerator (Electrostatics Corporation, Middleton WI) produces 0.5 to 5 MeV alpha particles that impinge on the sample. The energies of the backscattered particles are detected by a solid-state detector with an energy resolution of 15 keV. The energy is converted to depth using RUMP (Computer Graphic Service, Ithaca NY) software with a depth resolution ranging from 10 nm to 1 nm depending on the scattering geometry. The thickness and stoichiometry of the titanium oxide as well as impurities (~ atomic percent) can be determined without standards. The concentration and spatial distribution of N (amine), O (oxide) and C (APTS) will be determined by Auger Electron Spectroscopy (AES). AES spectra will be obtained with a Perkin-Elmer Phi 600 Scanning Auger multiprobe system. The sample is irradiated with 3keV electron beam at current of 0.1 mA. Lateral resolution is 100 nm. Surface charge will be evaluated with contact-angle goniometry. Static water contact angles will be measured using a home built contact angle goniometer. A 2 mL droplet of water will be suspended from the tip of a microliter syringe supported above the sample stage. The image of the droplet will be captured and the contact angle measured using ATI Multimedia and Scion Image programs.

RGD (SEQ ID NO: 1) Quantification. Peptide distribution will be quantitated in two ways. The first will be to use radiolabeled RGD (SEQ ID NO: 1). Briefly, RGD (SEQ ID NO: 1)-containing peptides will be radiolabeled with 125I. Radiolabeled RGD (SEQ ID NO: 1)-containing peptides will be incorporated into the surface as described above. Peptide distribution and density will be determined by autoradiography and by scintillation counting. The second method will be to use fluorescently labeled RGD (SEQ ID NO: 1). Briefly, commercially synthesized peptides will be produced with a fluorescent tag in place of the terminal chloroacetyl group. The peptides purchased from Sigma will be fluorescently labeled using the Alexa Fluor labeling kit (Molecular Probes, Eugene, OR). Peptides will be column separated and then resolved using ascending HETLC (72). Purified peptides will then be incorporated into the Ti discs as described above and quantitated in terms of density and distribution using confocal microscopy.

Detection of amount and density of acid-labile tigecyclin on Ti may be determined by spectrofluorimetry and by epifluorescence microscopy. Tethered tigecyclin will be imaged using its own inherent fluorescence. Quantities will be estimated by comparison with known amounts of tigecyclin in the fmol-nmol range. Digital images will be collected using a Noran Instruments confocal scanning laser microscope which we have successfully used in the past to image Ti surfaces. The confocality allows filtering of the out-of-phase reflected light from the metal surface.

The Ti- tigecyclin hybrids may be incubated in PBS (pH 5.0 - 8.0), weak acid, for 1-180 min. The PBS pH will be adjusted using the H₃PO₄:NaHPO₄ ratio, while holding ionic strength constant. The results of this test will indicate the most "active" pH's (for example pH 5.0, 5.5, 6.0 as well as physiological pH (7.4)) for release of the tethered molecules. The release may be determined in a time course where antibiotic release is measured at 1, 2, 3, and 24 h.

Bactericidal activity of tethered tigecyclin against *S. aureus* infections. The bactericidal activity of these surfaces will be tested against an initial inoculum of 10³ - 10⁶ *S. aureus,* a concentration that will occupy less than 1% of the 177 mm² modified Ti disk used for these experiments. Modified or unmodified discs will be sterilized by exposure to UV light and placed into the well of a 24-well tissue culture plate. 0.5 mL of DMEM containing 10% FBS will be added and medium will be inoculated with 10³ - 10⁶ infectious units of *S. aureus.* The inoculum will be mixed by agitation on a rotary shaker for 5 minutes, followed by incubation at 37°C under normal tissue culture conditions, i.e., in a 5% CO₂, humidified incubator with no agitation. Bacteria proliferation will be measured as a function of time (1-24 h). Control surfaces will test the proliferation of the bacteria on the Ti alone, the effect of soluble tigecyclin on bacterial propagation and the effect of the linker alone. The proliferation curve of the bacteria will be determined every 60 minutes for 4 h by extraction of the DNA and measurement using PCR. Non-adherent bacteria will be collected, pelleted, and resuspended in 100 mL PBS. Adherent bacteria on the surface will be bathed in 100 mL PBS. Cell lysis will be achieved by four freeze/thaw cycles, followed by centrifugation. The supernatants will be used for determination of bacterial number by PCR. Briefly, 10 - 20 mL of the above DNA solutions will be used in a 100 mL reaction volume using a Tris-KCI-MgCl₂ buffer and AmpliTaq, as known to those skilled in the art. Primers are directed at the 16S rRNA (Forward: CGGCAGGCCTAACACATGCAAGTCG. Reverse: GGTTGCGGCCGTACTCCCCAGG) to yield an 881 bp product, after 30 - 35 cycles. Detection will be by ethidium bromide staining after fractionation by agarose gel electrophoresis. In addition, pH measurements will be performed to determine if bulk changes in pH are occurring as a result of bacterial propagation. In parallel, bacterial number will be measured by direct counting after plating serial dilutions on agar plates. Supernatant will be removed from cultures containing *S. aureus* as above and the surfaces will be washed with 2 mL of DMEM and pooled with the supernatant from the same culture. Following agitation, this supernatant will be plated on LB agar plates at dilutions of 0, 102, 104, and 106 for determination of bacterial numbers. Additional dilutions will be used, as appropriate, to yield a countable number of colonies on the plate. Molecular Probes "Live/Dead BacLight" kit can be used to visualize bacterial viability based on differential staining. Using the Live/Dead BacLight system (Molecular Probes), live and dead bacteria will be stained with SYTO9 + propidium iodide. Live cells will fluoresce green, whereas cells with compromised membranes will fluoresce red. Digital images will be recorded and ratios of live:dead bacteria determined in random fields using double immunofluorescence.

Biocompatibility of the tethered tigecyclin. Biocompatibility will be determined by culturing MC3T3 osteoblast-like cells on the tethered antibiotic surface. MC3T3-E1 cells will be maintained in DMEM containing 10% FBS, 25 mg/mL ascorbate, and 5 mM β-glycerophosphate (for mineralization studies). At 1, 5, and 10 days, numbers of adherent cells will be determined by the BCECF-AM assay and actual cell numbers will be measured by DNA analysis. To assess development of the osteoblastic phenotype the expression of Osf-2, osteocalcin, and collagen type I will be evaluated by RT-PCR. The functional status of the cells at 7 and 14 days will be determined by measuring the activity of alkaline phosphatase. The mineralization potential will be evaluated by examining the cell layer for evidence of apatite formation by FT-IR.

Bactericidal activity and biocompatibility of tethered tigecyclin in presence of cells. The modified surface should be permissive to osteoblastic cell proliferation and only release antibiotic in the presence of bacteria. Based on the results of the studies described above, two concentrations of bacteria will be co-cultured with MC3T3-E1 osteoblast-like cells. Effects on osteoblast phenotype and function will be assessed as described, and bacterial survival will also be evaluated.

The outcome measures include the surface density of the modified tigecyclin tethered to Ti and the composition of the tigecyclin derivative that is attached to the surface. The overall efficiency of attachment of tigecyclin to the Ti surface may be 80-90%. As only one amide group has sufficient reactivity to be modified in the proposed reaction, it is expected that predominantly one reaction product, namely the one described herein will be formed. This prediction will be tested by MALDI-TOF-mass spectrometry. If other groups are derivatized then alternative protecting groups will be used to ensure that only that amide is available for reaction with the Ti-linker-terminal amine. After attachment of tigecyclin to the Ti surface, extensive washing procedures will be employed, making it unlikely that the surface will contain active, unmodified, untethered antibiotic. In the case of less than stoichiometric coupling, some linker alone will be found tethered onto the metal surface. Since it is expected that the treatments will result in 80-90% modification, the concentration of the unreacted linker will be small compared to the concentration of the modified tigecyclin. Should the actual density of the antibiotic on the Ti surface be non-uniform, then additional passivation techniques will be employed to ensure a uniform, fresh Ti surface. For the acid-labile linkage, it is expected that acidic solutions will cause significant and time-dependent release of tigecyclin. In the neutral pH range, there should be minimal release of the antibiotic. Should either acid labile linkage release too quickly, the linkage may be modified to decrease its electron density, making it less likely to be protonated. The linkages may be used to tether other antibiotics to the Ti surface, as new antibiotics are generated. These procedures result in a delivery system for the incorporation of bioactive substances into solid synthetic surfaces.

Bacterial proliferation should be retarded by the RGD (SEQ ID NO: 1)/antibiotic-modified surfaces. The determination of the concentrations of antibiotic needed to produce a bactericidal effect in an essentially two-dimensional system will be determined from the experiment described *vide supra.* Based on the antibiotic occupying ~40 nm² of space, if an adherent bacterium occupies 1 mm², it will have ~25,000 molecules of antibiotic tethered for release/interaction directly at its surface. Based on preliminary data these numbers of tethered antibiotics should be sufficient to eradicate a bactericidal infection. The experiments detailed allow measurement of the numbers of adherent and planktonic bacteria via PCR and colony formation assays, and also permit visualize the live to dead ratio on the surface and in solution using dyes specific for living or dead bacteria. If the number of tethered antibiotic molecules is inadequate, multivalent linkages via the RGD (SEQ ID NO: 1)/APTS bonds may be used to increase tigecyclin concentration.

The RGD (SEQ ID NO: 1) surface should enhance osteoblast maturation on the Ti surface. The addition of antibiotics to the peptide-grafted surface should not inhibit cellular adhesion or osteoblast maturation. This prediction is based on the fact that other antibiotic-containing surfaces that have much higher concentrations of antibiotics immobilized on their surfaces support such maturation. If the modified surface retards osteogenesis, different surface concentrations of the antibiotic will be used to increase ontogenesis.

The crystal structure of the RGD (SEQ ID NO: 1) loop region indicates that the motif is on an extended β hairpin-like loop within the fibronectin molecule. Based on this loop region, use of longer RGD (SEQ ID NO: 1) peptides may allow modification of the three dimensional configuration to further promotes cell adhesion and maturation. In this case, it may be able to determine the required length of the loop region that is optimum for osteoblast binding to the Ti.

### EXAMPLE 5

In this example the relationship between bacterial proliferation and bulk pH changes is used to illustrate that secretions from bacteria may be used as an indication of cellular activity and result in release of therapeutic molecules from a chemically modified surface.

To measure pH changes associated and bacterial proliferation, serum-free DMEM was inoculated with *E*. *coli* and the bacterial proliferation and solution pH monitored over the course of 6 hours at 37°C in a bacterial shaker. During this time, the bacteria were still in the log phase of growth and the pH of the solution decreased over 4 pH units, bringing the DMEM from its normal, slightly basic pH to a pH below 5.0. These results were reproducible. The acid released is an example of one of several environmental cues that can be exploited in designing implants with modified surfaces that release therapeutic molecules in response to the secretions of physiological state.

### EXAMPLE 6

In this prophetic example method and composition are described that may be used to couple a therapeutic molecule such as the antibiotic rifampin to an implant surface that includes a Ti-oxide surface. The coupling is further illustrated by using weak-acid-labile linkages. Method and procedures used to characterize such modified implant surfaces and their release kinetics are detailed.

Rifampin (C₄₃H₅₈N₄O₁₂, 822.95 Da) is an example of a large polycyclic antibiotic. The non-active 17-OH group of the antibiotic rifampin ((2S*, 12Z, 14E, 16R*, 17R*, 18S*, 19S*, 20S*, 21R*, 22S*, 23R*, 24E)-5, 6, 9,17,19, 21-hexahydroxy-23-methoxy-2, 4, 12, 16, 18, 20, 22-heptamethyl-8-[N-(4-methyl-1-piperazinyl)formimidoyl] 2,7-(epoxypentadeca[1,11,13]trienimino)naphtha[2,1-b]furan-1,11-deone 21-acetate) may be modified by esterification with. 2-methyl-4-thioethylamido maleic acid. This reaction may be monitored by TLC, using reactants as standards and the product characterized by HPLC and mass spectroscopy. The modified rifampin (sm-Rif) can be covalently attached to an activated, smooth Ti alloy surface via production of a Ti-S or through use of APTS chemistry to produce a Ti-O-SiR bonded group. After reaction, the antibiotic surface density on the Ti as well as the extent of immobilization may be characterized. The surface density may be measured using a top-down microtiter plate reader with fluorescent capabilities (amounts can be determined by comparison with known amount of rifampin dried onto a Ti surface). The distribution of the sm-Rif may be determined using eipfluorescence microscopy. Characterization of the moieties tethered to the surface can be determined by Matrix Assisted Laser Desoprtion Ionization-Time Of Flight (MALDI-TOF) mass spectroscopy. Characterization of the molecular ions resulting from the fragmentation of the sm-Rif can be compared with values obtained from unmodified rifampin and published literature values.

The surface linkage stability and release of a tethered molecule such sm-Rif from the Ti may be determined as a function of time and pH. Measurement of the effect of pH on the release rates of the antibiotic from the surface at different time periods can be made. The total solubilized rifampin released by the change in pH may be determined by spectrophotometry or spectrofluorimetry. Biological activity of the tethered molecules may be measured using a disc diffusion assay using a lawn of *S*. *aureus.* In this assay, active antibiotic on a surface derivatized or chemically modified implant produces a zone of growth inhibition around the disk and by comparison with known concentrations of antibiotic, produces an estimate of the active concentration of antibiotic on the surface of the implant.

### EXAMPLE 7

In this prophetic example, specific conditions for the modification of rifampin and attachment of the modified rifampin to a titanium disc are disclosed.

All synthetic reactions can be performed with sterile solutions to minimize subsequent UV sterilization prior to cell culture. Rifampin can be obtained from Sigma or from BIOMOL Research Laboratories, with an estimated purity of >95%.

Synthesis of smRif. 2-Thioethylamine can be reacted with 1.5 equivalents of 2-methyl maleic anhydride in dry CH₃CN with 0.1 equivalents of dimethylaminopyridine (DMAP) in a glove box with stirring under argon at 25°C until complete conversion to 2-methyl maleyl thioethylamide. Conversion can be measured by TLC on silica developed with 70% CHCl₃/30% MeOH. Unreacted 2-methyl maleic anhydride can be hydrolyzed by addition of aqueous NaHCO₃. The 2-methyl maleyl thioethylamide product can then be extracted twice from the solution with one volume of CHCl₃. The organic layer can be washed again with aqueous NaHCO₃, then added to two volumes of pentane to precipitate the 2-methyl maleyl thioethylamide product. The product can be characterized by TLC and mass spectroscopy. Product that is at least 90% pure can be esterified in dry CHCl₃ to the 17-OH of 0.5 equivalents of rifampin upon the addition of 1.2 equivalents of N,N'-dicyclohexylcarbodiimide (DCC) with stirring under argon at 25°C until complete conversion to 17-(2-methyl-4-thioethylamido maleyl) rifampin (sm-Rif), assayed by TLC on silica developed with 50% CHCl₃/50% MeOH. The resulting N,N'-dicyclohexylurea can be sedimented, and the solution can then be extracted with one volume of diethyl ether. The CHCl₃ phase can then be added to two volumes of pentane to precipitate the sm-Rif product. The product can be characterized by C₁₈ reversed-phase HPLC and MALDI-TOF mass spectroscopy.

HPLC monitoring of product formation. Sm-Rif can be purified from contaminating rifampin and 2-methyl maleyl thioethylamide by argon-flushed reversed-phase liquid chromatography on a 10x250 mm Alltima C₁₈ column eluted with a gradient over 30 min. from 10% CH₃CN/90% H₂O to 90% CH₃CN/10% H₂O at 2 mL/min, monitored at 238 nm.

A fresh Ti surface can be produced by incubation of smooth Ti disks in nitric acid to remove the oxide coating, followed by extensive rinsing with de-gassed, oxygen-free PBS in a nitrogen atmosphere. The sm-Rif, dissolved in oxygen-free PBS can be reacted with the fresh Ti surface in an inert atmosphere for 30 min. at 25°C. The thiol bond to the Ti is a favorable reaction that proceeds with high efficiency in the absence of oxygen. As a control, the 2-methyl maleyl thioethylamide precursor can also be reacted with a fresh Ti surface.

Alternatively the sm-Rif, may be coupled to a titanium surface silylated with (3-glycidoxypropyl)trimethoxysilane (GPTS) to form a thioether Ti-O-Si(CH₂OH)(CH₂)-Sm-RIF

Non-hydrolyzable Ti-Rif can be synthesized by a similar esterification route, except that 3-thiopropanoic acid can be condensed with the 17-OH of rifampin followed by purification and characterization as above. Bonding to the Ti surface can be as above.

Detection of amount and density of rifampin on Ti by spectrophotometry or fluorimetry. Rifampin can be detected by fluorimetry after oxidation. Either the surface containing the immobilized rifampin or the solution containing rifampin can be oxidized in 6% H₂O₂ in PBS for 5 min. An equal volume of PBS, pH 7.9 can then be added and incubation can continue for 25 min., at which time rifampin fluorescence is optimal. Fluorescence persists for approximately an additional 30 min, limiting the numbers of oxidations that can be performed at any one time. Oxidation and fluorimetric detection of rifampin on the surface can be used for determination of the amount of rifampin covalently bonded to the implant surface. Measurement of solubilized rifampin can either use fluorimetry as above, with sensitivity in the 0.1 µg/mL range, or spectrophotometry (λ=334 nm, ε=27000 g-mole/l).

MALDI-TOF Mass Spectroscopy. Derivatized Ti disks overlaid with a sinapinic acid matrix can be allowed to outgas under high vacuum in the Ciphergen sample chamber for 30 min. before analysis. Molecular ion formation can be achieved through firing of a 338 nm laser onto the surface of the Ti, causing bond breakage and release of the parent ion. Because of the innate charge of the molecular ions, the parent ion and any fragments can be accelerated into the mass spectrometer where the time of flight can be a function of the mass of the molecular ions. Computer assignment of the identities of molecular ions by Ti-smRif can be based upon reported values for rifampin and comparison with the pattern produced by underivatized rifampin alone.

The Ti-smRif hybrids can be incubated in PBS (pH 5.0-8.0) for 1-180 min. The PBS pH can be adjusted using the H₃PO₄: NaHPO₄ ratio, while holding ionic strength constant. The most "active" pH values, which are expected to be pH 5.0, 5.5 and 6.0 for the present linkage, but which may be different for other linkages, may be determined in a time course where antibiotic release can be measured at 1, 2, 3 and 24 h. In parallel, the surface can also be incubated in PBS, pH 7.4 to determine the release rate under normal physiological pH.

Disc Diffusion Assay. Six mm sterile filter disks can be directly placed onto a full-grown lawn of *S*. *aureus* (ATCC 25923) culture, and known volumes (20 µL) and dilutions of the eluted antibiotic can be pipetted onto the disc. After incubation overnight, the area of the resultant adjacent clear zone that is devoid of bacterial growth can be measured and compared with the area cleared by a standard series of known doses of rifampin. A 1 µg/mL solution of rifampin should give a clear zone of 4-5 cm. The lower limit of sensitivity of this assay is expected to be in the 10 ng/mL range.

After attachment of the antibiotics to the Ti surface, extensive washing procedures can be employed, making it unlikely that the surface can contain active, unmodified, untethered antibiotic. Measurement of active antibiotic released under mildly acidic conditions from modified implant substrates may be measured with a disk diffusion assay using S. *Aureus* as the target bacteria.

### EXAMPLE 8

In this prophetic example, procedures, methods, and compositions used to determine if antibiotic release from compositions of the present invention lowers bacterial counts while sustaining osteoblastic cell proliferation and maturation are described.

In the establishment of an infection, the number of bacteria present at the site is initially very low. The size of the initial inoculum in these experiments can be determined by two parameters. Sufficient bacteria to be able to estimate bacterial number over several hours using PCR should be present. Using the PCR conditions known to those skilled in the art, it is possible to measure bacterial numbers greater than 1 x 10³. If the numbers of bacteria present are only sufficient to coat a small area of the surface, it may be possible to assess the effects of the surface modifications on a pre-monolayer. Assuming a size of 1-5 µm² for bacteria, 10³-10⁶ bacteria would occupy less than 1% of the total area of the disk for a 24-well plate and therefore should be within the test target. Inoculation will be made using about 10³-10⁶ *S*. *aureus* into 0.5 mL DMEM containing 10%FBS.

Using this inoculum of cells, the bactericidal activity of the Ti-smRif surface or other functionalized surfaces can be tested. Three control surfaces, unmodified Ti ± 1 µg/mL Rif (in the bathing medium), Ti-modified with the linker alone (Ti-sm), and Ti-modified with irreversibly-bonded rifampin (Ti-Rif) can be tested in parallel with the Ti-smRif using DMEM containing 10%FBS as the bathing medium. The control surfaces can test the proliferation of the bacteria on the Ti alone, the effect of soluble Rif on bacterial propagation, the effect of the linker alone (which is the moiety that can be left after bond cleavage) on osteoblast and bacterial proliferation, and finally the effect of irreversibly immobilized rifampin on bacterial proliferation, respectively. The proliferation curve of the bacteria over the course of about 4 hours can be determined every 60 minutes by extraction of the DNA and measurement using PCR. Numbers of bacteria can be estimated by comparison with PCR signals using DNA isolated from known numbers of bacteria. In addition, pH measurements can be performed as a result of bacterial propagation. Bacterial number can also be measured by direct counting. Serial dilutions of the bacteria can be made and plated onto LB agar. Colony formation can be assessed by cell counting after photography and total numbers of colonies calculated. These values can be compared with the numbers determined by PCR. This comparison can ensure that the numbers determined in the PCR experiments reflect bacteria capable of further proliferation (or under optimal conditions, represent a true cure of the bacterial infection).

Using two concentrations of bacteria that will be killed when incubated with the active surface, the effects of co-culture with Saos-9 osteoblast-like cells can be determined. These cells can be plated at a density of 1 x 10⁴ cells/1.77 cm² disk at the same time as inoculation of bacteria. It can then determine if the Ti-smRif or other bonded antibiotic surface impedes the bacteria's adhesion, proliferation, and maturation. Measurement of osteoblast number and expression of the osteoblastic cell phenotype may be continued for about 14 days so that functional indicators, including the activity of alkaline phosphatase and mineral formation can be evaluated. Control conditions can include osteoblastic cells on the Ti and Ti-sm surfaces.

### EXAMPLE 9

In this prophetic example specific procedures and conditions that may be used to determine if antibiotic release from modified implant surface of the present invention lowers bacterial counts while sustaining osteoblastic cell proliferation and maturation.

Modified or unmodified discs can be sterilized by exposure to UV light and placed into the well of a 24-well tissue culture plate. 0.5 mL of DMEM containing 10% FBS can be added and medium can be inoculated with probably 10³-10⁶ infectious units of S. *aureus.* The inoculum can be mixed by agitation on a rotary shaker for 5 minutes, followed by incubation at 37°C under normal tissue culture conditions, i.e. in a 5% CO₂, humidified incubator with no agitation. Bacteria proliferation can be measured as a function of time (1-24 h).

Non-adherent bacteria can be gathered in the supernatant, pelleted, and resuspended in 100 µL PBS. Adherent bacteria on the surface can be bathed in 100 µL PBS. Cell lysis can be achieved by four freeze/thaw cycles. Freezing can be in drylice ethanol and thawing at 65°C, for 1 min for each cycle. DNA from the adherent samples can be transferred to an Eppendorf tube, and the disk further rinsed with an additional 100 µL of PBS to remove any DNA still adherent to the surface. After the freeze/thawing cycles, the samples can be centrifuged in a microfuge for two minutes and the supernatants can be used for determination of bacterial number by PCR.

PCR may be used to determine bacterial number. Briefly, 10-20 µL of the above DNA solutions can be used in a 100 µL reaction volume using a Tris-KCl-MgCl₂ buffer and AmpliTaq. Primers are directed at the 16S rRNA (Forward: CGGCAGGCCTAACACATGCAAGTCG. Reverse: GGTTGCGGCCGTACTCCCCAGG) to yield an 881 bp product. PCR conditions are as follows: denaturation: 94°C, 1 min. Annealing: 55°C, 1 min. Extension: 72°C, 2 min for 30-35 cycles. Detection can be by ethidium bromide staining after fractionation by agarose gel electrophoresis.

Determination of *S*. *aureus* numbers by dilution. Supernatant can be removed from cultures containing *S*. *aureus* and the surfaces can be washed with 2 mL of DMEM and pooled with the supernatant from the same culture. Following agitation, this supernatant can be plated on LB agar plates at dilutions of 0, 10², 10⁴, and 10⁶ for determination of bacterial numbers. Additional dilutions can be used, as appropriate, to yield a countable number of colonies on the LB surface. Surfaces can be photographed and cell numbers determined by direct cell counting. Determinations can be performed by two independent investigators.

Determination of Saos-9 cell adhesion, proliferation, and maturation. The Ti can be harvested, washed three times in PBS to remove bacteria. The cells can be released from the Ti surface by treatment with 0.05% trypsin for 5 min, and collected by centrifugation at 200 x g for 10 min. Cells can be washed and resedimented three times. This procedure will remove almost all of the adherent bacteria. Numbers of adherent cells can be determined by the BCECF-AM assay. Numbers of the viable Saos-9 cells can be determined by MTT assay; actual cell numbers can be measured by DNA analysis. To assess development of the osteoblastic phenotype, we can evaluate the expression of Osf-2, osteocalcin, and collagen type I by RT-PCR. The functional status of the cells at 5, 10, and 14 days can be determined by measuring the activity of alkaline phosphatase. The mineralization potential can be evaluated by including 5 mM β-glycerophosphate in the culture medium and by examining the cell layer for evidence for apatite formation by FTIR.

### EXAMPLE 10

This example illustrates the immobilization of bioactive peptides to metal surfaces that can be bonded to other therapeutic molecules and that can result in osteoblast cell maturation due to the RGD (SEQ ID NO: 1) amino acid sequence.

The peptide RGD (SEQ ID NO: 1) was couple to surfaces using the silane surface chemistry. The impact of the peptide coupling on the surface was examined using atomic force microscopy and by the effect of the peptide on the differentiation of attached osteoblasts. The atomic force micrographs illustrate that the treatment causes a small change in surface roughness of the substrate. It was observed that cells were immediately bound to the membrane and proliferated. After 8 days on the material surface, osteoblasts exhibited high levels of alkaline phosphatase staining, indicating that the cells were undergoing maturation. Alizarin red staining and Fourier transform infrared (FTIR) spectroscopy showed that the mineral formed by the cells was a biological apatite. Control cells, maintained on the tripeptide RGE, did not exhibit these effects within the time period evaluated. This example demonstrates that even though the peptide tether contains only three amino acids, it retains its bioactivity on the material surface.

### EXAMPLE 11

This prophetic example demonstrates preparation and characterization of a surface with therapeutic molecules covalently bonded to an implant surface that enhances bone cell attachment and thereby facilitates tissue-implant integration, while remaining a long-term reservoir of therapeutic oligonucleotides to eradicate any recurrence of osteosarcoma or other cancers that arise locally or from hematogenous sources, i.e., metastatic cells.

For enhanced osseointegration, the RGD (SEQ ID NO: 1) peptide motif can be used as in Example 4 above. The implant can include covalently linked RGD (SEQ ID NO: 1)-containing peptides to a Ti surface, creating a peptide-Ti hybrid. This can provide a mixed surface as some of the RGD (SEQ ID NO: 1) motifs will serve as part of the linker for the immobilization of the therapeutic oligonucleotides. The RGD (SEQ ID NO: 1)-oligonucleotide linkage will be labile to matrix metalloproteinases released by cancer cells, thereby exposing additional RGDs (SEQ ID NO: 1) for promotion of osseointegration as the therapeutic oligonucleotide is released. Peptide sequences specifically cleaved by matrix metalloproteinases will be placed between the RGD (SEQ ID NO: 1) motif and the therapeutic oligonucleotide. The effect of such flanking amino acid sequences may be determined by progressively increasing the flanking amino acids at each side of the RGD (SEQ ID NO: 1) motif and anchor on the modified peptides covalently bonded to the Ti surface.

Therapeutic oligonucleotides targeted against the MYC oncogene constitute one example of a broad anticancer oligonucleotide. The therapeutic oligonucleotides may be composed of various combinations of modified bases, modified sugars, and modified internucleotide linkages. The therapeutic oligonucleotide targeted against the MYC oncogene may include peptide nucleic acid (PNA) residues linked to Ti-O-Si(CH₂)₃NH-RGD(SEQ ID NO: 1) by a peptide sequence specifically cleaved by matrix metalloproteinases, and a basic peptide sequence conducive to cellular uptake. The metalloproteinase-susceptible linker will be cleaved by matrix metalloproteinases secreted by cancer cells growing at the site of the implant. The liberated basic peptide-PNA chimera will be taken up quickly into the cytoplasm of the cancer cells, where it will inhibit the translation of MYC mRNA. As a result, production of Myc protein will be significantly reduced. Because Myc protein plays a vital role in cell proliferation, growth of the immediately neighboring cancer cells will be reduced.

Ti alloy (Ti6Al4V) surfaces for the implant will be obtained, modified, and characterized as described in Example 4.

The modified Ti surfaces can be evaluated for oligonucleotide release kinetics in the presence of osteosarcoma cells in culture, and for antiproliferative activity against the osteosarcoma cells. The modified surface may also be characterized to determine if the modified surface supports osteoblast proliferation and maturation both in the presence and absence of metastatic cancer cells.

## Claims

1. An implantable article comprising:
an implant for a mammal having a biologically compatible surface, wherein at least a portion of said surface is silylated with organosilanes;
a long, flexible and partially membrane-soluble linker having linking groups wherein a terminus of a linking group is covalently bonded to one or more of said organosilanes and wherein said linker is at least one of oligo(ethylene glycol) and an acid labile bond; and
at least one therapeutic molecule, wherein said therapeutic molecule is bonded to said linking group or linking groups and wherein said therapeutic molecule interacts with cells adjacent to the surface of said implant.

2. The implantable article of claim 1 wherein said therapeutic molecule enters a membrane of said cells or passes through a wall of said cells.

3. The implantable article of claim 1 wherein the therapeutic molecule is a member selected from the group consisting of peptide, therapeutic oligonucleotides, an antibiotic, cell growth factors, chemotherapeutics, thrombolytic, anti-inflammatories, and osteoactive factors.

4. The implantable article of claim 1 wherein said therapeutic molecule includes a peptide.

5. The implantable article of claim 4 wherein labilization of the therapeutic molecule from the linker leaves a peptide covalently bonded to the surface that promotes the adhesion and maturation of cells.

6. The implantable article of claim 1, where the linker includes oligo(ethylene glycol).

7. The implantable article of claim 1 wherein the implant is made from at least one of titanium, titanium alloy, tantalum, CoCrMo alloys, stainless steels, cobalt, chromium, aluminum, zirconium, gold, silicon and their alloys, TEFLON/PTFE, polyethylene, ultra high molecular weight polyethylene, silicone, silica, glass, polystyrene and zirconia.

8. The implantable article of claim 1 wherein said linker includes the acid labile bond, and said therapeutic molecule is released from said linker by interacting with said cells.

9. The implantable article of claim 1 wherein the therapeutic molecule interacts with said cells to alter angiogenesis, or decrease bacterial proliferation adjacent to said implant.

10. The implantable article of claim 1 wherein said therapeutic molecule is cleaved from said linker by intracellular enzymes or acid.

11. The implantable article of claim 1 wherein said therapeutic molecule is exchanged with endogenous ligands of the cell.

12. The implantable article of claim 1 wherein said acid labile linker is a member selected from the group consisting of methylmaleamide, hydrazone, and combinations thereof.

13. The implantable article of claim 3 wherein labilization of the antibiotic from the linker provides the peptide that promotes the adhesion and maturation of bone cells.

14. The implantable article of claim 3 wherein the antibiotic is a member selected from the group consisting of minocyclins, tigecyclin, glycylcycline, vancomycin and its analogs, rifampin and its analogs, gentamycin and its analogs, or combinations thereof.

15. The implantable article of claim 3 wherein said implant promotes osseointegration.

16. The implantable article of claim 3 wherein said therapeutic molecule is a peptide and an antibiotic and wherein the antibiotic is competitively bonded to said linker.

17. The implantable article of claim 8 wherein said therapeutic molecule includes a peptide.

18. The implantable article of claim 1 wherein said linker comprises aminoethoxyethoxyacetate (AEEA) units.

19. The implantable article of claim 1 wherein said linker comprises 2-20 repeat units.

20. The implantable article of claim 1 wherein said implant is at least one of intramedullary nail and a prosthesis.

## Patentansprüche

1. Implantierbarer Gegenstand umfassend:
ein Implantat für einen Säuger, welches eine biologisch kompatible Oberfläche aufweist, wobei mindestens ein Teil der Oberfläche mit Organosilanen silyliert ist;
einen langen, flexiblen und partiell membran-löslichen Linker mit Linkergruppen, wobei ein Ende einer Linkergruppe an eines oder mehrere der Organosilane kovalent gebunden ist und wobei der Linker mindestens eines von Oligo(ethylenglycol) und einer säure-labilen Bindung ist; und
mindestens ein therapeutisches Molekül, wobei das therapeutische Molekül an die Linkergruppe oder Linkergruppen gebunden ist und wobei das therapeutische Molekül mit Zellen in der Nähe der Oberfläche des Implantats wechselwirkt.

2. Implantierbarer Gegenstand nach Anspruch 1, wobei das therapeutische Molekül in eine Membran der Zellen gelangt oder eine Wand der Zellen passiert.

3. Implantierbarer Gegenstand nach Anspruch 1, wobei es sich bei dem therapeutischen Molekül um ein Element handelt, welches aus der Gruppe, die aus einem Peptid, therapeutischen Oligonukleotiden, einem Antibiotikum, Zellwachstumsfaktoren, Chemotherapeutika, Thrombolytika, anti-inflammatorischen Mitteln und osteoaktiven Faktoren besteht, ausgewählt ist.

4. Implantierbarer Gegenstand nach Anspruch 1, wobei das therapeutische Molekül ein Peptid einschließt.

5. Implantierbarer Gegenstand nach Anspruch 4, wobei die Labilisierung des therapeutischen Moleküls von dem Linker ein kovalent an die Oberfläche gebundenes Peptid zurücklässt, welches die Adhäsion und Reifung von Zellen fördert.

6. Implantierbarer Gegenstand nach Anspruch 1, wobei der Linker Oligo(ethylenglycol) einschließt.

7. Implantierbarer Gegenstand nach Anspruch 1, wobei das Implantat aus mindestens einem von Titan, Titanlegierung, Tantal, CoCrMo-Legierungen, rostfreien Stählen, Kobalt, Chrom, Aluminium, Zirconium, Gold, Silicium und deren Legierungen, Teflon/PTFE, Polyethylen, Polyethylen mit ultrahohem Molekulargewicht, Silicon, Siliciumdioxid, Glas, Polystyrol und Zirconiumdioxid hergestellt ist.

8. Implantierbarer Gegenstand nach Anspruch 1, wobei der Linker die säure-labile Bindung einschließt und das therapeutische Molekül von dem Linker durch Wechselwirkung mit den Zellen freigesetzt wird.

9. Implantierbarer Gegenstand nach Anspruch 1, wobei das therapeutische Molekül mit den Zellen wechselwirkt, um die Angiogenese zu beeinflussen oder die Bakterienproliferation in der Nähe des Implantats herabzusetzen.

10. Implantierbarer Gegenstand nach Anspruch 1, wobei das therapeutische Molekül von dem Linker durch intrazelluläre Enzyme oder Säure abgespalten wird.

11. Implantierbarer Gegenstand nach Anspruch 1, wobei das therapeutische Molekül gegen endogene Liganden der Zelle ausgetauscht wird.

12. Implantierbarer Gegenstand nach Anspruch 1, wobei es sich bei dem säure-labilen Linker um ein Element handelt, welches aus der Gruppe, die aus Methylmaleamid, Hydrazon und Kombinationen davon besteht, ausgewählt ist.

13. Implantierbarer Gegenstand nach Anspruch 3, wobei die Labilisierung des Antibiotikums von dem Linker das Peptid bereitstellt, welches die Adhäsion und Reifung von Knochenzellen fördert.

14. Implantierbarer Gegenstand nach Anspruch 3, wobei es sich bei dem Antibiotikum um ein Element handelt, welches aus der Gruppe, die aus Minocyclinen, Tigecyclin, Glycylcyclin, Vancomycin und dessen Analoga, Rifampin und dessen Analoga, Gentamycin und dessen Analoga, oder Kombinationen davon besteht, ausgewählt ist.

15. Implantierbarer Gegenstand nach Anspruch 3, wobei das Implantat die Osseointegration fördert.

16. Implantierbarer Gegenstand nach Anspruch 3, wobei das therapeutische Molekül ein Peptid und ein Antibiotikum ist und wobei das Antibiotikum kompetitiv an den Linker gebunden ist.

17. Implantierbarer Gegenstand nach Anspruch 8, wobei das therapeutische Molekül ein Peptid einschließt.

18. Implantierbarer Gegenstand nach Anspruch 1, wobei der Linker Aminoethoxyethoxyacetat(AEEA)-Einheiten umfasst.

19. Implantierbarer Gegenstand nach Anspruch 1, wobei der Linker 2-20 Wiederholungseinheiten umfasst.

20. Implantierbarer Gegenstand nach Anspruch 1, wobei das Implantat mindestens eines von einem intramedullären Nagel und einer Prothese ist.

## Revendications

1. Article implantable comprenant :
un implant pour un mammifère ayant une surface biologiquement compatible, dans lequel au moins une partie de ladite surface est silylée avec des silanes organiques ;
un long lieur souple et partiellement soluble dans une membrane ayant des groupes de liaisons dans lequel un terminus du groupe de liaisons est lié de manière covalente à un ou plusieurs desdits silanes organiques et dans lequel ledit lieur est au moins l'un parmi un oligo(éthylène glycol) et une liaison labile en milieu acide ; et
au moins une molécule thérapeutique, dans lequel ladite molécule thérapeutique est liée audit groupe de liaisons ou aux groupes de liaisons et dans lequel ladite molécule thérapeutique interagit avec les cellules adjacentes à la surface dudit implant.

2. Article implantable selon la revendication 1, dans lequel ladite molécule thérapeutique pénètre dans une membrane desdites cellules ou traverse une paroi desdites cellules.

3. Article implantable selon la revendication 1, dans lequel la molécule thérapeutique est un élément choisi dans le groupe comprenant les peptides, les oligonucléotides thérapeutiques, un antibiotique, les facteurs de croissance cellulaire, les agents chimiothérapeutiques, les agents thrombolytiques, les anti-inflammatoires et les facteurs ostéoactifs.

4. Article implantable selon la revendication 1, dans lequel ladite molécule thérapeutique comprend un peptide.

5. Article implantable selon la revendication 4, dans lequel la labilisation de la molécule thérapeutique provenant du lieur laisse un peptide lié de manière covalente sur la structure, ce qui favorise l'adhérence et la maturation des cellules.

6. Article implantable selon la revendication 1, dans lequel le lieur comprend un oligo(éthylène glycol).

7. Article implantable selon la revendication 1, dans lequel l'implant est réalisé à partir d'au moins l'un parmi le titane, l'alliage de titane, le tantale, les alliages CoCrMo, les aciers inoxydables, le cobalt, le chrome, l'aluminium, le zirconium, l'or, la silicone et leurs alliages, le TEFLON/PTFE, le polyéthylène, le polyéthylène à poids moléculaire ultra élevé, le silicium, la silice, le verre, le polystyrène et la zircone.

8. Article implantable selon la revendication 1, dans lequel ledit lieur comprend la liaison labile en milieu acide, et ladite molécule thérapeutique est libérée dudit lieur en interagissant avec lesdites cellules.

9. Article implantable selon la revendication 1, dans lequel la molécule thérapeutique interagit avec lesdites cellules pour modifier l'angiogenèse, ou diminuer la prolifération bactérienne à proximité dudit implant.

10. Article implantable selon la revendication 1, dans lequel ladite molécule thérapeutique est séparée dudit lieur par des enzymes intracellulaires ou un acide.

11. Article implantable selon la revendication 1, dans lequel ladite molécule thérapeutique est échangée par des ligands endogènes de la cellule.

12. Article implantable selon la revendication 1, dans lequel ledit lieur labile en milieu acide est un élément choisi dans le groupe comprenant le méthyl-maléimide, l'hydrazone et leurs combinaisons.

13. Article implantable selon la revendication 3, dans lequel la labilisation de l'antibiotique du lieur fournit le peptide qui favorise l'adhérence et la maturation des cellules osseuses.

14. Article implantable selon la revendication 3, dans lequel l'antibiotique est un élément choisi dans le groupe comprenant les minocyclines, la tigécycline, les glycylcyclines, la vancomycine et leurs analogues, la rifampine et ses analogues, la gentamycine et ses analogues, ou leurs combinaisons.

15. Article implantable selon la revendication 3, dans lequel ledit implant favorise l'intégration osseuse.

16. Article implantable selon la revendication 3, dans lequel ladite molécule thérapeutique est un peptide et un antibiotique et dans lequel l'antibiotique est lié de manière compétitive audit lieur.

17. Article implantable selon la revendication 8, dans lequel ladite molécule thérapeutique comprend un peptide.

18. Article implantable selon la revendication 1, dans lequel ledit lieur comprend des unités de ((aminoéthyl)éthoxy acétyl) (AEEA).

19. Article implantable selon la revendication 1, dans lequel ledit lieur comprend des unités de répétition 2-20.

20. Article implantable selon la revendication 1, dans lequel ledit implant est au moins l'un parmi un clou intramédullaire et une prothèse.


### SEQUENCE LISTING

<110> Thomas Jefferson University
   Noreen, Hickok J.
   Eric, wickstrom
<120> ADVANCED BIOMATERIALS AND METHODS OF ATTACHING THERAPEUTIC AGENTS THERETO
<130> 100051.00102
<140> NOT YET ASSIGNED
   <141> 2004-09-14
<150> 10/662,261
   <151> 2004-09-15
<150> 10/662,261
   <151> 2003-09-15
<160> 6
<170> PatentIn version 3.2
<210> 1
   <211> 3
   <212> PRT
   <213> Artificial
<220>
   <223> Bonded to an organosilane
<400> 1
   Arg Gly Asp
   1
<210> 2
   <211> 4
   <212> PRT
   <213> Artificial
<220>
   <223> Bonded to an organosilane
<400> 2
   Arg Gly Asp Ser
   1
<210> 3
   <211> 4
   <212> PRT
   <213> Artificial
<220>
   <223> Bonded to an organosilane
<400> 3
   Arg Gly Glu Ser
   1
<210> 4
   <211> 4
   <212> PRT
   <213> Artificial
<220>
   <223> Bonded to an organosilane
<400> 4
   Asp Gly Glu Ala
   1
<210> 5
   <211> 4
   <212> PRT
   <213> Artificial
<220>
   <223> Bonded to an organosilane
<400> 5
   Glu Gly Glu Ala
   1
<210> 6
   <211> 30
   <212> PRT
   <213> Artificial
<220>
   <223> Bonded to an organosilane
<400> 6
   Pro Gly Val Asp Tyr Thr Ile Thr Val Tyr Ala Val Thr Gly Arg Gly
   1               5                   10                  15
   Asp Ser Pro Ala Ser Ser Lys Pro Val Ser Ile Asn Tyr Arg
               20                  25                  30